# EUROPEAN PATENT SPECIFICATION

(11) **EP 2 313 509 B1**
(45) Date of publication and mention of the grant of the patent: **04.09.2013**
(21) Application number: 09794064.7
(22) Date of filing: 07.07.2009
(51) Int. Cl.: C12N 15/82

(54) **PLANTS PRODUCING 2N POLLEN**
2N-POLLEN-PRODUZIERENDE PFLANZEN
PLANTES PRODUISANT DU POLLEN 2N

(30) Priority: 08.07.2008 EP 08290672
(43) Date of publication of application: 27.04.2011
(73) Proprietor: Institut National De La Recherche Agronomique, 75007 Paris (FR)
(72) Inventor: MERCIER, Raphael, 78330 FONTENAY LE FLEURY (FR); D'ERFURTH, Isabelle, 21800 Quetigny (FR); CROMER, Laurence, 92240 Malakoff (FR)
(74) Representative: Vialle-Presles, Marie José
(86) International application number: PCT/IB2009/006590
(87) International publication number: WO 2010/004431

(56) References cited:
- SCHOLL RANDY L ET AL: "Seed and molecular resources for Arabidopsis" PLANT PHYSIOLOGY (ROCKVILLE), vol. 124, no. 4, December 2000 (2000-12), pages 1477-1480, XP002505485 ISSN: 0032-0889
- DATABASE UniProt [Online] 1 June 2001 (2001-06-01), "SubName: Full=Putative uncharacterized protein F7P12.4; DTYITTMEIS VPLASPNVLT LARDSIKTQK LQSTQDFQTP TMWDLDVVEA AAEKPSSSCV" XP002505487 retrieved from EBI accession no. UNIPROT:Q9C8N3 Database accession no. Q9C8N3
- MGLINETS A V ET AL: "Inheritance of the ps mutation in sugar beet" RUSSIAN JOURNAL OF GENETICS, vol. 43, no. 5, May 2007 (2007-05), pages 525-529, XP002505486 ISSN: 1022-7954
- RAMANNA M S ET AL: "Relevance of sexual polyploidization for crop improvement: A review" EUPHYTICA, KLUWER ACADEMIC PRESS, AMSTERDAM, NL, vol. 133, no. 1, 1 January 2003 (2003-01-01), pages 3-18, XP002381902 ISSN: 0014-2336 cited in the application

### SEQUENCE LISTING

<110> INSTITUT NATIONAL DE LA RECHERCHE AGRONOMIQUE MERCIER, Raphael D'ERFURTH, Isabelle
<120> Plants producing 2n pollen
<130> MJPhzF539/139-WO
<160> 40
<170> PatentIn version 3.3
<210> 1
   <211> 1477
   <212> PRT
   <213> Arabidopsis thaliana
<400> 1
   Met Glu Val Lys Glu Glu Lys Leu Met Glu Glu Lys Gln Arg Leu Pro
   1               5                   10                  15
   Glu Lys Thr Ile Pro Val Phe Thr Val Leu Lys Asn Gly Ala Ile Leu
               20                  25                  30
   Lys Asn Ile Phe Val Val Asn Ser Arg Asp Phe Ser Ser Pro Glu Arg
           35                  40                  45
   Asn Gly Ser Thr Val Ser Asp Asp Asp Gly Glu Val Glu Glu Ile Leu
       50                  55                  60
   Val Val Gly Arg His Pro Asp Cys Asp Ile Leu Leu Thr His Pro Ser
   65                  70                  75                  80
   Ile Ser Arg Phe His Leu Glu Ile Arg Ser Ile Ser Ser Arg Gln Lys
                   85                  90                  95
   Leu Phe Val Thr Asp Leu Ser Ser Val His Gly Thr Trp Val Arg Asp
               100                 105                 110
   Leu Arg Ile Glu Pro His Gly Cys Val Glu Val Glu Glu Gly Asp Thr
           115                 120                 125
   Ile Arg Ile Gly Gly Ser Thr Arg Ile Tyr Arg Leu His Trp Ile Pro
       130                 135                 140
   Leu Ser Arg Ala Tyr Asp Leu Asp Asn Pro Phe Val Ser Pro Leu Asp
   145                 150                 155                 160
   Ala Ser Thr Val Leu Glu Gln Glu Glu Glu Asn Arg Met Leu Glu Ala
                   165                 170                 175
   Glu Asn Leu Glu Val Ala Gln His Gln Ser Leu Glu Asn Thr Thr Ser
               180                 185                 190
   Gly Asp Glu Gly Val Leu His Leu Asp Val Thr Ser Glu Gly Thr Gly
           195                 200                 205
   Ser Ser Val Pro Ser Glu Asp Glu Asp Thr Tyr Val Thr Thr Arg Glu
       210                 215                 220
   Met Ser Met Pro Val Ala Ser Pro Ser Val Leu Thr Leu Val Arg Asp
   225                 230                 235                 240
   Ser Val Glu Thr Gln Lys Leu Gln Phe Asn Glu Asp Leu Gln Thr Ser
                   245                 250                 255
   Pro Lys Trp Asp Leu Asp Val Ile Glu Ser Val Ala Glu Lys Leu Ser
               260                 265                 270
   Gly Ser Phe Val Arg Ser Thr Gln Gln Ser Gly Gly Asp Val Glu Gly
           275                 280                 285
   Leu Gly Cys Ser Glu Leu Phe Asp Ala Ala Glu Ala Asp Glu Cys Asp
       290                 295                 300
   Val Arg Gly Asp Gly Gly Leu His Leu Asn Val Ile Ser Glu Lys Met
   305                 310                 315                 320
   Glu Ser Ser Val Pro Asn Met Ile Glu Ala Glu Asn Leu Glu Val Ala
                   325                 330                 335
   Gln His Gln Ser Leu Ala Asn Thr Ala Leu Gly Asp Asp Glu Asp Leu
               340                 345                 350
   His Leu Asp Val Thr Ser Glu Gly Thr Gly Ser Ser Val Pro Ser Glu
           355                 360                 365
   Asp Glu Asp Thr Tyr Ile Thr Thr Met Glu Ile Ser Val Pro Leu Ala
       370                 375                 380
   Ser Pro Asn Val Leu Thr Leu Ala Arg Asp Ser Ile Lys Thr Gln Lys
   385                 390                 395                 400
   Leu Gln Ser Thr Gln Asp Phe Gln Thr Pro Thr Met Trp Asp Leu Asp
                   405                 410                 415
   Val Val Glu Ala Ala Ala Glu Lys Pro Ser Ser Ser Cys Val Leu Gly
               420                 425                 430
   Lys Lys Leu Ser Gly Gly Tyr Val Glu Glu Leu Gly Cys Phe Glu Leu
           435                 440                 445
   Phe Val Ala Ala Glu Ala Asp Lys Cys Asp Val Arg Gly Asp Gly Ser
       450                 455                 460
   Leu His Leu Asn Glu Ile Ser Glu Arg Met Glu Ser Ser Met Ser Asn
   465                 470                 475                 480
   Lys Glu Asp Asp Pro Phe Leu Ala Ala Lys Glu Thr Ser Ser Leu Pro
                   485                 490                 495
   Leu Ser Thr Asp Phe Ile Asn Pro Glu Thr Leu Trp Leu Val Glu Asp
               500                 505                 510
   Val Gln Ala Ser Pro Glu Phe Cys Thr Ser Ser Val Lys Ala Asn Ala
           515                 520                 525
   Glu Asn Pro Ser Ser Gly Cys Ser Pro Ser Thr Glu Gln Ile Asp Gly
       530                 535                 540
   Cys Phe Glu Thr Ser Gly Cys Ser Ala Phe Asp Leu Ala Ala Glu Val
   545                 550                 555                 560
   Glu Ser Leu Ser Leu His Gln Glu Val Ser Glu Glu Thr Glu Phe Val
                   565                 570                 575
   Thr Lys Glu Val Met Gly Val Ser Ser Glu Pro Leu Gly Lys Ala Asp
               580                 585                 590
   Ile Arg Ser His Glu Glu Asn Gly Glu Ser Glu Asp Ser Arg Gln Val
           595                 600                 605
   Ile Glu Val Ser Ala Glu Pro Val Ala Lys Ala Asp Ile Gln Ser His
       610                 615                 620
   Glu Glu Asn Gly Glu Thr Glu Gly Ser Arg Gln Val Ile Glu Val Ser
   625                 630                 635                 640
   Pro Lys Ser Phe Ser Glu Ala Glu Pro Thr Ile Glu Ile Leu Thr Gly
                   645                 650                 655
   Glu Ala Gln Gly Ile Ile Gly Ser Glu Phe Pro Ser Glu Leu Ala Val
               660                 665                 670
   Glu Thr Glu Ser Glu Asn Leu Leu His Gln Lys Ser Ile Gly Glu Thr
           675                 680                 685
   Lys Asn Glu Ile Arg Ser His Glu Asp Tyr Gly Glu Thr Glu Asp Tyr
       690                 695                 700
   Gly Glu Thr Glu Cys Ser Trp Pro Asp Ile Ala Val Ser Pro Ser Ser
   705                 710                 715                 720
   Val Ser Pro Pro Glu Pro Thr Leu Glu Ile Leu Thr Asp Glu Ala Arg
                   725                 730                 735
   Gly Leu Leu Gly Ser Glu Phe Leu Ser Glu Val Thr Val Glu Thr Glu
               740                 745                 750
   Ile Glu Asn Leu Leu His Gln Lys Ser Asn Val Glu Thr Lys Ala Asp
           755                 760                 765
   Ile Leu Ile His Glu Asp Tyr Gly Glu Thr Glu Val Ser Arg Gln Ile
       770                 775                 780
   Ile Thr Val Ser Pro Asn Ser Phe Ser Lys Ala Glu Pro Thr Leu Glu
   785                 790                 795                 800
   Thr Glu Asp Ser Arg Gln Gln Ala Arg Gly Leu Val Gly Ser Asp Ser
                   805                 810                 815
   Glu Phe Gln Ser Glu Val Ala Met Lys Thr Glu Cys Glu Asn Leu Leu
               820                 825                 830
   Asn Gln Lys Arg Asn Gly Glu Thr Lys Val Ser Ser Arg Gln Ala Ser
           835                 840                 845
   Pro Val Ser Asp Cys Leu Ser Thr Pro Lys Asp Arg Leu Ser Ser Ile
       850                 855                 860
   Asn Thr Asp Asp Ile Gln Ser Leu Cys Ser Ser Ser Gln Pro Pro Ser
   865                 870                 875                 880
   Glu Ser Glu Val Asn Pro Ala Thr Asp Gln Asp Gln Glu Ser Gly Ile
                   885                 890                 895
   Ile Ser Glu Thr Glu Lys Pro Lys Thr Glu Leu Leu Ile Gly Ser Gly
               900                 905                 910
   Arg Ser Glu Lys Tyr Tyr Ser Leu Ser Glu Ile Glu Gly Glu Glu Asn
           915                 920                 925
   Thr Asp Ile Gly Arg Leu Ser Arg Cys Pro Ile Pro Ser Ala Leu Ala
       930                 935                 940
   Ala Lys Thr Ser Glu Asp Thr Lys Leu Ile Glu Glu Leu Ser Ser Ser
   945 950 955 960
   Asp Ser Gly Ser Gln Glu Asn Gln Thr Pro Glu Thr His Ala Val Arg
   965 970 975
   Asp Asp Val Leu Cys Asp Met Asp Ser Ser Ser Thr Cys Asn Ile Trp
   980 985 990
   Ser Arg Arg Gly Lys Ala Ala Ser Val Leu Lys Ile Arg Thr Asn Lys
   995 1000 1005
   Ser Gln Gly Lys Gln Lys Gln Thr Gly Arg Gln Pro Lys Asp Lys
   1010 1015 1020
   Leu His Arg Lys Gln Ala Leu Ser Asp Lys Ser Ile Ser Leu Thr
   1025 1030 1035
   Ile His His Gly Ala Glu Ile Leu Glu Pro Glu Ile Phe Thr Pro
   1040 1045 1050
   Asp Lys Glu Asn Leu Thr Pro Ser Ser His Met Leu Lys Arg Leu
   1055 1060 1065
   Gln Asp Ile Gly Asp Val Lys Asp Ser Lys Ser Ser Leu Lys Leu
   1070 1075 1080
   Ser Gly Lys Ser Cys Ser Ser Leu Val His Ser Ser Ile Ala Val
   1085 1090 1095
   Leu Ala Ser Glu Ala Phe Thr Glu Pro Glu Ile Phe Thr Pro Asp
   1100 1105 1110
   Lys Glu Asn Leu Thr Pro Ser Ser His Met Leu Lys Arg Leu Arg
   1115 1120 1125
   Glu Phe Gly Asp Ile Lys Asp Thr Lys Gly Ser Ser Ser Lys Ala
   1130 1135 1140
   Thr Arg Lys Pro Phe Phe Asp Ile Arg Met Glu Glu Asn Val Met
   1145 1150 1155
   Val Glu Gln Glu Pro Glu Asp Leu His Ser Leu Gly Ser Lys Ser
   1160 1165 1170
   Lys Leu Lys His Glu Pro Leu Ala Pro Lys Lys Lys Ala Glu Arg
   1175 1180 1185
   Ala Pro Phe Gln Pro Leu Leu Glu Lys Ser Ser Phe Gln Ser Gln
   1190 1195 1200
   Ser Tyr Thr Glu Ala Ser Ser Thr Ala Ser Ala Arg Asn Asn Ile
   1205 1210 1215
   Ser Arg Gly Ile Arg Ser Ser Ser Asn Leu Ser Asp Ala Lys Ser
   1220 1225 1230
   Lys Met Lys Trp Thr Ile Val Leu Asp Thr Ser Ser Leu Leu Asp
   1235 1240 1245
   Lys Glu Ser Arg Lys Pro Leu Gln Leu Leu Gln Gly Leu Lys Gly
   1250 1255 1260
   Thr His Leu Val Val Pro Arg Thr Val Leu Arg Glu Leu Asn Glu
   1265 1270 1275
   Val Lys Arg Ser Arg Ser Phe Leu Phe Arg Arg Arg Thr Glu Ile
   1280 1285 1290
   Ala Ser Ser Ala Leu Asp Trp Ile Glu Glu Cys Lys Val Asn Ser
   1295 1300 1305
   Lys Trp Trp Ile Gln Val Gln Ser Pro Thr Glu Glu Thr Lys Ala
   1310 1315 1320
   Ile Ala Pro Thr Pro Pro Val Thr Pro Gln Ser Asn Gly Ser Ser
   1325 1330 1335
   Ala Phe Pro Phe Ser Leu His Trp Asn Asn Tyr Ala Pro Glu Ile
   1340 1345 1350
   Asp Ser Pro Thr Ser Glu Asp Gln Val Leu Glu Cys Ala Leu Leu
   1355 1360 1365
   Tyr Arg Asn Arg Asn Arg Asp Glu Lys Leu Val Leu Leu Ser Asn
   1370 1375 1380
   Asp Val Thr Leu Lys Ile Lys Ala Met Ala Glu Gly Val Ile Cys
   1385 1390 1395
   Glu Thr Pro His Glu Phe Tyr Glu Ser Leu Val Asn Pro Phe Ser
   1400 1405 1410
   Glu Arg Phe Met Trp Thr Glu Ser Thr Ala Arg Gly Arg Thr Trp
   1415 1420 1425
   Ser His Leu Asp Asn Asp Val Leu Arg Glu Arg Tyr Asn Asp Arg
   1430 1435 1440
   Ala Cys Arg Arg Lys Ser Thr Tyr Asn Arg Gly Glu Ser Gly Ala
   1445 1450 1455
   Ala Ala Lys Gly Leu Lys Leu Ile Leu Leu His Asn Ser His Tyr
   1460 1465 1470
   Gly His Thr His
   1475
<210> 2
   <211> 1079
   <212> PRT
   <213> Populus trichocarpa
<400> 2
   Met Ala Ser Asn Glu Glu Lys Lys Pro Glu Glu Glu Glu Glu Glu Glu
   1               5                   10                  15
   Arg Lys Ile Pro Val Phe Thr Val Leu Arg Asn Gly Ala Ile Leu Lys
               20                  25                  30
   Asn Ile Phe Val Ile Asp Lys Ser Pro Leu Pro Ser Pro Thr Ser Ser
           35                  40                  45
   Glu Pro Ser Ile Glu Asn Glu Glu Asn Pro Val Gln Glu Thr Glu Glu
       50                  55                  60
   Ile Leu Ser Phe Gly Arg His Pro Asp Cys Ser Ile Val Leu Asn His
   65                  70                  75                  80
   Pro Ser Ile Ser Arg Phe His Leu Gln Ile Asn Ser Arg Pro Ser Ser
                   85                  90                  95
   Gln Lys Leu Phe Val Thr Asp Leu Ser Ser Val His Gly Thr Trp Val
               100                 105                 110
   Ser Gly Lys Lys Ile Glu Pro Gly Phe Arg Val Glu Leu Asn Glu Gly
           115                 120                 125
   Asp Thr Ile Arg Val Gly Gly Ser Thr Arg Tyr Tyr Arg Leu His Trp
       130                 135                 140
   Val Pro Leu Ser Arg Ala Tyr Asp Met Glu Thr Pro Phe Ile Ser Pro
   145                 150                 155                 160
   Leu Asp Met Ala Met Ile Glu Glu Lys Arg Glu Glu Asn Pro Val Leu
                   165                 170                 175
   Glu Glu Glu Asn Glu Ala Lys Met Ser Gln Asp Glu Asn Leu Val Ala
               180                 185                 190
   Thr Glu Arg Glu Ser Val Glu Glu Lys Gly Ser Leu Glu Val Ala Gly
           195                 200                 205
   Lys Asp Asp Glu Arg Tyr Gln Ala Met Asp Ser Thr Ser Val Glu Asn
       210                 215                 220
   Arg Glu Thr Lys Ser Leu Asp Leu Ile Leu Gln Asp Val Gly Ser Leu
   225                 230                 235                 240
   Tyr Cys Glu Glu Ile Cys Glu Ser Ile Ala Lys Lys Glu Ile Leu Ser
                   245                 250                 255
   Ala Ala Leu Val Pro Asp Glu Ser Met Asp Ser Leu Phe Tyr Asp Ala
               260                 265                 270
   Asn Glu Asp Ile Glu Ile Ser Phe Arg Asn Asp His Asn Val Lys Asp
           275                 280                 285
   Ile Leu Ser Pro Thr Thr Val Gln Gly Val Ile Ser Glu Thr Lys Cys
       290                 295                 300
   Arg Gln Tyr Asp Gly His Asn Gln Ser Pro Glu Tyr Phe Ser Val Arg
   305                 310                 315                 320
   Gln Glu Leu Pro Glu Thr Glu Thr Lys Gly Ser Ser Met Ile Arg Glu
                   325                 330                 335
   Ser Asn Ala Val Phe Ser Ser Leu Ser Thr Ala Glu Val Glu Ser Gln
               340                 345                 350
   Ser Ala Ser Glu Met Leu Gly Ala Thr Glu Asn Gly Ser Leu Leu Arg
           355                 360                 365
   Lys Gly His Glu Pro Ile Asn Ile Phe Ser His Gly Ile Glu Met Val
       370                 375                 380
   Asn Leu Ser Leu Pro Val Lys Asp Leu Ser Glu Asn Asp Ser Lys Lys
   385                 390                 395                 400
   Val Arg Lys Glu Asn Gln Thr Leu Glu His Leu Val Ala Leu Glu Pro
                   405                 410                 415
   Thr Tyr Lys Glu Gly Asn Gln Gly Asn Phe Thr Ala Asn Leu Leu Val
               420                 425                 430
   Asn Leu Asn Ser Ala Cys Ser Asp Asp Gln Ala Val Ser Leu Lys Ser
   435 440 445
   Thr Tyr Glu Glu Gly Asn Gln Glu Lys Phe Thr Ala Asn Leu Leu Glu
   450 455 460
   Asn Leu Asn Ser Ala Cys Ser Gly Asp His Ala Val Ala Leu Asp Pro
   465 470 475 480
   Thr Tyr Glu Glu Gly Thr Arg Glu His Phe Thr Ala Asn Leu Leu Glu
   485 490 495
   Asn Leu Asn Thr Ser Ser Cys Ser Asp Asp His Ala Val Ala Leu Asp
   500 505 510
   Pro Thr Tyr Glu Glu Gly Thr Gln Glu Asn Phe Thr Thr Asn Leu Leu
   515 520 525
   Glu Asn Leu Asn Ser Ser Cys Ser Asp Asp His Ala Ala Asp Asp Met
   530 535 540
   Leu Glu Val Glu Asn Gln Asn Leu Ser Arg Asp Asp His Gly Gln Ser
   545 550 555 560
   Val Tyr Thr Ser Ile Cys Ser Ala Leu Leu Ala Ala Glu Ser Val Ser
   565 570 575
   Ser Ser Phe Pro Val Gly Leu Leu Ser Glu Ile Ile Asp Ser Lys Lys
   580 585 590
   Cys Gln Thr Pro Glu Ser Val Leu Ala Ser Ile Glu Asn Gln Glu Asn
   595 600 605
   Leu Gln Ser Ser His Val Arg Ser Glu Lys Lys Gln Ser Ser Arg Asn
   610 615 620
   Ile Trp Ser Arg Arg Gly Lys Pro Lys Ala Val Leu Gln Leu Gln Thr
   625 630 635 640
   Ser Arg Ser Arg Glu Lys Asn Arg Gly Asp Asp Val Glu Trp Glu Asn
   645 650 655
   Gln Glu Asn Ile Glu Asn Arg Ser Ile Ser Lys Thr Ile Phe Pro Gly
   660 665 670
   Ser Glu Ala Ala Glu Glu Val Leu Thr Pro Gly Lys Glu Asn Tyr Ser
   675 680 685
   Pro Asn Thr Leu Leu Leu Lys Ser Leu Lys Lys Lys Gly Lys Arg Glu
   690 695 700
   Glu Thr Gln Leu Ser Asn Ser Arg Arg Ser Thr Ser Ser Lys Ile Ala
   705 710 715 720
   Phe Ser Pro Tyr Lys Gln Pro Glu Glu Glu Met Ile Ala Ser Pro Asp
   725 730 735
   Lys Glu Asn Gln Thr Pro Lys Val Leu Gln Gln Thr Lys Leu Ala Ile
   740 745 750
   Pro Ala Ser Arg Asn Gln Val Lys Phe Lys Gln Glu Met Val Leu Glu
   755 760 765
   Glu Cys Lys Ala Glu Arg Val Pro Leu Gln Ser Leu Leu Val Asn Phe
   770 775 780
   Ser Gly Asn Ser Asn Ser Glu Ala Ser Val Pro Asn Asp Ala Thr Arg
   785 790 795 800
   Ser Ser Ile Ser Val Asn Cys Ser Gln Ile Met Arg Lys Ser Asn Phe
   805 810 815
   Thr Gly Asp Gly Lys Arg Arg Trp Thr Met Val Ala Asp Thr Ala Ser
   820 825 830
   Leu Val Asp Lys Glu Ser Arg Lys Ser Leu Gln Leu Leu Gln Gly Leu
   835 840 845
   Lys Gly Thr His Leu Val Ile Pro Lys Met Val Ile Arg Glu Leu Asp
   850 855 860
   Cys Leu Lys Arg Arg Ser Ser Leu Phe Arg Lys Lys Thr Glu Ala Ser
   865 870 875 880
   Leu Val Leu Glu Trp Ile Glu Glu Cys Met Val Arg Thr Pro Trp Trp
   885 890 895
   Ile His Val Gln Ser Ser Met Glu Glu Gly Arg His Ile Ala Pro Thr
   900 905 910
   Pro Pro Ala Ser Pro Gln Ser Arg Phe Ser Gln Gly Ser Glu Gly Phe
   915 920 925
   Pro Cys Gly Thr Gly Ser Ser Val Pro Phe Pro Ala His Gly Ser Phe
   930 935 940
   Leu Glu Ile Val Ser Pro Thr Ala Glu Asp His Ile Leu Glu Tyr Ala
   945 950 955 960
   Leu Ser Tyr Arg Lys Met Asn Arg Asp Gly Gln Leu Ile Leu Leu Thr
   965 970 975
   Asn Asp Val Thr Leu Lys Ile Lys Ala Met Ser Glu Gly Leu Ile Cys
   980 985 990
   Glu Thr Ala Lys Glu Cys Arg Asp Ser Leu Val Asn Pro Phe Ser Glu
   995 1000 1005
   Arg Phe Leu Trp Ala Asp Ser Ser Pro Arg Gly Gln Thr Trp Ser
   1010 1015 1020
   Val Ser Asp Asp Leu Val Leu Lys Glu Arg Tyr Tyr Gln Ser Pro
   1025 1030 1035
   Ser Lys Lys Ser Ser Lys Gly Glu Gly Ala Lys Gly Leu Lys Leu
   1040 1045 1050
   Ile Leu Leu His Asn Ser Gln Tyr Gly Gln Ile Ser Arg Ser Glu
   1055 1060 1065
   Gln Cys Ser Leu Phe Arg Asn Arg Tyr Leu Phe
   1070 1075
<210> 3
   <211> 1112
   <212> PRT
   <213> Oryza sativa
<400> 3
   Met Ala Ser Ala Ala Ala Glu Gly Glu Glu Ala Pro Ile Ala Ala Phe
   1               5                   10                  15
   Ala Val Ser Lys Gly Gly Val Val Leu Lys Asn Ile Phe Leu Asn Ala
               20                  25                  30
   Pro Pro Ser Pro Leu Pro Val Glu Glu Ala Ala Arg Gly Arg Gly Gly
           35                  40                  45
   Glu Glu Glu Asp Pro Pro Val Met Phe Gly Arg His Pro Glu Cys His
       50                  55                  60
   Val Leu Val Asp His Pro Ser Val Ser Arg Phe His Leu Glu Val Arg
   65                  70                  75                  80
   Ser Arg Arg Arg Gln Arg Arg Ile Thr Val Thr Asp Leu Ser Ser Val
                   85                  90                  95
   His Gly Thr Trp Ile Ser Gly Arg Arg Ile Pro Pro Asn Thr Pro Val
               100                 105                 110
   Glu Leu Thr Ala Gly Asp Val Leu Arg Leu Gly Ser Ser Arg Arg Glu
           115                 120                 125
   Tyr Arg Leu His Trp Leu Ser Leu Pro Glu Ala Phe Asp Met Glu Asp
       130                 135                 140
   Leu Leu Pro Pro Leu Leu Glu Glu Asp Lys Glu Glu Leu Ser Thr Cys
   145                 150                 155                 160
   Gln Glu Ala Ser Lys Gln Leu Glu Pro Asp Gln Lys Glu Ser Ala Asp
                   165                 170                 175
   Thr Glu Thr His Gln Glu Thr Ser Gln Gln Val Val Ser Glu Gln Ile
               180                 185                 190
   Asp Phe His Ala Asn Val Ile Pro Ser Ala Pro Pro Ile Pro Glu Phe
           195                 200                 205
   Ala Asp Leu Phe Ala Leu Glu Glu Ser Ser Val Pro Glu Phe Asp Asp
       210                 215                 220
   Ser Arg Glu Gly Arg Ile Glu Gly Asn Leu Ile Glu Glu Asn His Val
   225                 230                 235                 240
   Ile Tyr Ser Val Glu Ser Ser Ile Thr Gln Pro Met Leu Ala Thr Val
                   245                 250                 255
   Glu Asp Ala Gly Arg Ser Val Lys Ser Gly Glu Lys Asp Thr Ser Asn
               260                 265                 270
   Ala Arg Arg Ser Lys Leu Lys Ser Val Lys Thr Leu Arg Ile Glu Thr
           275                 280                 285
   Gly Arg Ser Lys Glu Arg Ile Thr Pro Leu Ser Tyr Ser Tyr Gln Lys
       290                 295                 300
   Glu Glu Asn Gln Asn Glu Asn Pro Ile Cys Ser Gln Asn Cys Gly Ile
   305                 310                 315                 320
   Glu Cys Glu Ala Cys Met Val Leu Phe Asn Asn Ser Tyr Val Gly Glu
                   325                 330                 335
   Ala Glu Glu Lys Glu Lys Met Asn Ile Leu Asp Arg Ile Met Met Glu
   340 345 350
   Glu Asn Gln Glu Gln Thr Asn His Leu Gln Ser Lys Glu Phe Val His
   355 360 365
   Tyr Val Ala Pro Leu Asn Leu Asp Tyr Glu Thr Phe Ser Asp Asn Glu
   370 375 380
   Asn Cys Val Leu Ser Val Ala Lys Glu Thr Glu His Asn Asp Phe Asn
   385 390 395 400
   Ser Val Asn Cys Ile Ser Gln Asp Ser Val Cys Glu Asn Pro Gln Lys
   405 410 415
   Ile Ser Glu Leu Leu His Phe Val Ser Pro Leu Val Phe Lys Gly Asp
   420 425 430
   Asp Phe Thr Asp Ser Lys Ile Leu Gln Leu Cys Ala Ser Val His Lys
   435 440 445
   Glu Leu Ser Gly Pro Ile Leu Glu Asn Pro Phe Met Gln Asp Ile Ser
   450 455 460
   Asp Glu Asn Thr Asn Ser Asn Lys Asp Thr Gly His Glu Gly Leu Thr
   465 470 475 480
   Leu Leu Asn Leu Asp Ala Thr Leu Thr Ser Asn Glu Asn Phe Ala Gln
   485 490 495
   Ser Lys Ile Phe Val Ala Pro Glu Asp Ser Glu Ser Glu Gly Thr Ile
   500 505 510
   Ser Glu Asn Leu Phe Glu Ile Ser Asn Met Lys Gly Asn Glu Glu Asn
   515 520 525
   Glu Glu Asn Ser Pro Trp Asp Lys Glu Asn Ile Thr Pro Phe Val Ser
   530 535 540
   Gly Asp Ile Ile Val Glu Arg Ser Gln Leu Arg Leu Lys Pro Thr Thr
   545 550 555 560
   Ile Ser Gln Glu Leu Met Asp Ser Ile Ser Pro Leu Asn Leu Glu His
   565 570 575
   Asn Asp Phe Ser Asp Asp Glu Ser Ser Ile Leu Ser Ile Gly Glu Gln
   580 585 590
   Met Asn Ser Asn Glu Leu Ile Ala Lys Asn Leu Ile Pro Leu Thr Ser
   595 600 605
   Val Asp Ala Asn Met Gln Lys Ser His Ala Gly Phe Met Thr Ile Ala
   610 615 620
   His Leu Asp Phe Lys Asp Ser Ile Leu Thr Asp Glu Glu Thr Ser Val
   625 630 635 640
   Leu Ser Pro Glu Lys Tyr Asp Thr Ile Ser Pro Val Arg Gln Gly Asn
   645 650 655
   Leu Phe Pro Asp Lys Glu Asn Val Thr Pro Ala Ser Arg Asp Leu Lys
   660 665 670
   Pro Ile Ile Gly Arg Lys Val Leu Gly Pro Arg Val Asp Asn Ser Leu
           675                 680                 685
   Ser Val Glu Cys Thr Ser Lys Arg Arg Ile His Arg Gln Glu Pro Asn
       690                 695                 700
   Glu Leu Ser Ala Lys Ser Lys Val Cys His Ala Val Asp Asp Asp Val
   705                 710                 715                 720
   Phe Tyr Ser Asp Lys Glu Asn Leu Thr Pro Ile Ser Ser Gly Gly Ile
                   725                 730                 735
   Lys Ala Arg Arg Cys Leu Pro Lys Ser Leu Thr Val Asp Ala Asp Gln
               740                 745                 750
   Asp Gln Glu Ala Phe Tyr Ser Asp Lys Glu Asn Leu Thr Pro Val Ser
           755                 760                 765
   Ser Ala Ser Arg Lys Thr Lys Asp Leu Ser Glu Asn Arg Ala Arg Met
       770                 775                 780
   Glu Ser Thr Ile Thr Lys Lys Arg Val Val Asp Arg Leu Pro Phe Gln
   785                 790                 795                 800
   Thr Leu Leu Ser Asn Ser Pro Leu Arg His Thr Ser Ser Leu Asp Ser
                   805                 810                 815
   Thr Gln Val Asn Pro Arg Ala Val Asp Val Ala Met Lys Leu Glu Gly
               820                 825                 830
   Glu Leu Asn Asn Val Pro His Lys Gly Gln Glu Ser Glu Lys Thr Lys
           835                 840                 845
   Glu Gly Met Lys Val Trp Thr Met Val Thr Asp Met Glu Cys Leu Leu
       850                 855                 860
   Asp Asp Glu Ser Arg Lys Ser Ile Met Leu Leu Arg Gly Leu Lys Gly
   865                 870                 875                 880
   Thr Gln Leu Val Ile Pro Met Ile Val Ile Arg Glu Leu Glu Cys Leu
                   885                 890                 895
   Lys Lys Arg Glu Arg Leu Phe Arg Met Leu Ser Lys Ala Thr Ser Met
               900                 905                 910
   Leu Gln Trp Ile Asn Glu Cys Met Glu Lys Glu Ser Trp Trp Ile His
           915                 920                 925
   Val Gln Ser Ser Thr Glu Met Leu Pro Val Ala Pro Thr Pro Pro Ala
       930                 935                 940
   Thr Pro Thr Ala Leu Cys Asn Asn Gly Glu Arg Glu Ile Ser Ala Gly
   945                 950                 955                 960
   Thr Phe Asn Pro Ile Ala Leu Phe Ser Pro Arg Ser Phe Ser Asp Ile
                   965                 970                 975
   Val Ser Pro Lys Thr Glu Asp Arg Val Leu Asp Cys Ala Leu Leu Phe
               980                 985                 990
   Asn Lys Leu Lys Gly Asn Gln Asn Ile Val Ile Leu Ser Asn Ser Val
           995                 1000                1005
   Thr Leu Lys Ile Lys Ala Met Ala Glu Gly Phe Pro Cys Glu Gly
       1010                1015                1020
   Ala Lys Glu Phe Arg Glu Thr Leu Val Asn Pro Cys Ser Ser Arg
       1025                1030                1035
   Phe Met Trp Ala Ala Ser Ala Pro Arg Gly Ser Ala Trp Ser Cys
       1040                1045                1050
   Leu Asp Glu Thr Thr Leu Glu Glu Asn Tyr Tyr Asn Ser His His
       1055                1060                1065
   Gly Ala Arg Arg Arg Ile Pro Arg Pro Met Glu Pro Ala Lys Gly
       1070                1075                1080
   Leu Lys Leu Ile Leu Leu His Asn Ser His Tyr Gly Gln Ala Thr
       1085                1090                1095
   Asn Phe Val Glu Asn Arg Pro Leu Ala Pro Met Ala Ser Trp
       1100                1105                1110
<210> 4
   <211> 869
   <212> PRT
   <213> Vitis vinifera
<400> 4
   Met Ala Asp Glu Asn Glu Lys Lys Ile Pro Val Phe Thr Val Leu Lys
   1               5                   10                  15
   Asn Asn Ala Ile Leu Lys Asn Ile Phe Val Ile Asp Gln Pro Pro Pro
               20                  25                  30
   Gly Ile Ser Glu Pro Glu Arg Pro Glu His Val Leu Glu Glu Ile Leu
           35                  40                  45
   Met Val Gly Arg His Pro Asp Cys Asn Ile Met Leu Thr His Pro Ser
       50                  55                  60
   Ile Ser Arg Phe His Leu Gln Ile Tyr Ser Asn Pro Thr Leu Gln Lys
   65                  70                  75                  80
   Leu Ser Val Met Asp Leu Ser Ser Val His Gly Thr Trp Val Ser Glu
                   85                  90                  95
   Lys Lys Ile Gln Pro Arg Ala Arg Val Glu Leu Lys Glu Gly Asp Ile
               100                 105                 110
   Ile Arg Leu Gly Ser Ser Ser Arg Ile Tyr Arg Leu His Trp Val Pro
           115                 120                 125
   Leu Ser Gln Ala Tyr Asp Leu Glu Asn Pro Phe Val Ser Ala Ser Asp
       130                 135                 140
   Val Leu Met Glu Glu Glu Lys Glu Asp Glu Ile Tyr Gln Asp Val Ser
   145                 150                 155                 160
   Ser Phe Ser Val Asp Ser Lys Glu Ile Gln Ser Gln His Pro Val Leu
                   165                 170                 175
   Lys Gly Met Glu Ser Val Phe Ser Asp Glu Asn Cys Glu Pro Phe Val
               180                 185                 190
   Glu Lys Pro Ile Pro Ser Ala Pro Pro Glu Pro Glu Asn Met Asn Ser
           195                 200                 205
   Ser Ala Ser Asp Glu Glu Lys Thr Glu Gly Glu Gly Leu Pro Val Val
   210 215 220
   Glu Ala Phe Glu Glu Ile Glu Asn Gln Ser Pro Ser Arg Arg Asp Tyr
   225 230 235 240
   Glu Gln Thr Glu Ile Leu Gly Ala Val Asn Leu Leu Pro Ser Ala Glu
   245 250 255
   Val Leu Leu Glu Thr Arg Asn Glu Gln Leu Asp Glu Glu Ile Lys Ser
   260 265 270
   Pro Gln Pro Leu Phe Val Ser Glu Val Phe Ser Gln Gly Glu Thr Pro
   275 280 285
   Val Gly Leu Pro Thr Lys Ser Trp Gln Lys Ser Lys Leu Leu Gly Ser
   290 295 300
   Leu Asp Ser Tyr Val Ala Asp Asp Lys Ile Glu Ile Pro Leu Val Ala
   305 310 315 320
   Glu Val Leu Glu Glu Val Glu Asn Gln Ser Pro Pro Arg Lys Gly Tyr
   325 330 335
   Glu Gln Arg Glu Ala Ser Gly Leu His Ser Gly Ala Ile Thr Thr Glu
   340 345 350
   Ser Val Asn Ser Ser Val Pro Asp Arg Asn Ile Leu Ser Asp Ile Gly
   355 360 365
   Asn Gln Gln Phe Ser Asn Glu Asn Gln Pro Pro Lys Pro Leu Pro Val
   370 375 380
   Thr Leu Gly Leu Ser Asp Asp Glu Asn Pro Glu Ser Pro Pro Val Arg
   385 390 395 400
   Leu Glu Gln Lys Ser Ser Leu Pro Asn Ile Trp Ser Arg Arg Gly Lys
   405 410 415
   Pro Ala Ser Val Leu Gln Ile Gln Thr Gly Arg Ser Thr Arg Lys Cys
   420 425 430
   Ile Gly Asp Gly Asn Gly Ala Lys Ile Arg Lys Pro Lys Gln Glu Asp
   435 440 445
   Leu Glu Asn Lys Pro Ile Ser Arg Ala Leu Phe Pro Met Leu Asp Gly
   450 455 460
   Glu Glu Thr Glu Ile Phe Thr Pro Asn Lys Glu Asn Phe Ser Pro Asn
   465 470 475 480
   Thr Leu Leu Leu Lys Ser Val Asn Lys Lys Lys Gly Ile Leu Glu Glu
   485 490 495
   Thr Lys Gln Ser Thr Leu Cys Arg Ser Ser Ser Ser Lys Phe Ser Thr
   500 505 510
   Gly Pro Asn Lys Cys Ser Glu Glu Asp Thr Ser Thr Phe Ser Asp Lys
   515 520 525
   Glu Asn Gln Thr Pro Gln Val Leu Gln Thr Arg Lys Ser Val Arg Pro
   530 535 540
   Ser Pro Glu Asn Ser Ser Arg Asn Arg Gly Lys Leu Glu Lys Glu Ile
   545 550 555 560
   Met Val Met Lys Arg Gly Ala Glu Arg Val Pro Phe His Ser Leu Leu
   565 570 575
   Glu Asn Ala Ala Cys Lys Ser Lys Ser Glu Val Ser Ile Leu Gly Ala
   580 585 590
   Lys Thr Arg Ser Ser Asn Ser Val Asn Cys Thr Gly Thr Thr Gly Asn
   595 600 605
   Ala Thr Asn Ser Ser Phe Asn Asn Ser Ala Gly Glu Gly Lys Arg Arg
   610 615 620
   Trp Asn Met Val Val Asp Ala Thr Cys Leu Leu Asn Lys Glu Ser Arg
   625 630 635 640
   Lys Ser Leu Gln Leu Leu Gln Gly Leu Lys Gly Thr Gln Leu Ile Ile
   645 650 655
   Pro Arg Met Val Ile Arg Glu Leu Asp Cys Leu Lys Arg Arg Gly Ser
   660 665 670
   Leu Phe Arg Arg Ile Ser Glu Val Ser Leu Val Leu Gln Trp Ile Glu
   675 680 685
   Glu Cys Met Val Lys Thr Lys Trp Trp Ile His Val Gln Ser Ser Ile
   690 695 700
   Glu Glu Gly Arg Pro Ile Ala Pro Thr Pro Pro Ala Ser Pro Pro Arg
   705 710 715 720
   Phe Ser Glu Gly Ser Gly Gly Phe Ile Ser Gly Thr Thr Ser Ser Val
   725 730 735
   Pro Phe Ser Ala Cys Gly Ser Leu Met Glu Ile Val Ser Pro Thr Ala
   740 745 750
   Glu Asp His Ile Leu Glu Cys Ala Leu Phe Phe Arg Arg Ile Lys Asn
   755 760 765
   Asp Gly Gln Leu Val Leu Phe Thr Asn Asp Val Thr Leu Lys Ile Lys
   770 775 780
   Ala Met Ala Glu Gly Leu Asn Cys Glu Thr Val Glu Glu Phe Arg Glu
   785 790 795 800
   Ser Leu Val Asn Pro Phe Ser Glu Arg Phe Met Trp Ser Asp Ser Ser
   805 810 815
   Pro Arg Gly Gln Thr Trp Ser Tyr Leu Asp Asp Val Val Leu Arg Glu
   820 825 830
   Lys Tyr Tyr Arg Cys Pro Leu Lys Lys Ala Ser Lys Gly Gly Glu Ser
   835 840 845
   Ala Lys Gly Leu Lys Leu Ile Leu Leu His Asn Ser His Tyr Gly Lys
   850 855 860
   Ile Gly Ser Ile Ser
   865
<210> 5
   <211> 1123
   <212> PRT
   <213> Glycine max
<220>
   <221> misc_feature
   <222> (700)..(700)
   <223> Xaa can be any naturally occurring amino acid
<400> 5
   Met Ala Glu Lys Lys Asn Pro Glu Gln Glu Glu Gln His Arg Phe Pro
   1               5                   10                  15
   Val Leu Thr Val Leu Lys Asn Asn Ala Ile Leu Lys Asn Ile Phe Ile
               20                  25                  30
   Val Leu Asp Glu His Asp Glu Asp Gln Thr Val Leu Ile Gly Arg His
           35                  40                  45
   Pro Asn Cys Asn Ile Val Leu Thr His Pro Ser Val Ser Arg Phe His
       50                  55                  60
   Leu Arg Ile Arg Ser Asn Pro Ser Ser Arg Thr Leu Ser Leu Val Asp
   65                  70                  75                  80
   Leu Ala Ser Val Gln Gly Thr Trp Val Arg Gly Arg Lys Leu Glu Pro
                   85                  90                  95
   Gly Val Ser Val Glu Leu Lys Glu Gly Asp Thr Phe Thr Val Gly Ile
               100                 105                 110
   Ser Thr Arg Ile Tyr Arg Leu Ser Trp Ala Pro Leu Thr Gln Leu Gly
           115                 120                 125
   Val Val Val Pro Gln Gln His Gln Lys Glu Asp Glu Gln Glu Asn Ile
       130                 135                 140
   Ile Lys Asp Glu Asn Leu Glu His Thr Ala Glu Gln Asp Ile Pro Met
   145                 150                 155                 160
   Ser Glu Asp Ile Val Ser Val Cys Cys Asp Glu Glu Arg Lys Ile His
                   165                 170                 175
   Ser Glu Asp Glu Ala Leu Gly Val Pro Asn Gly Thr Glu Thr Ser Cys
               180                 185                 190
   Phe Pro Thr Asn Ser Cys Val Glu Asn Ile Ile Cys Asp Cys Gln Leu
           195                 200                 205
   Ser Pro Pro Tyr Ile Gln Ser Pro Pro Cys Ala Gln Pro Val Asp Glu
       210                 215                 220
   Leu Asp Asn Thr Lys Lys Ile Glu Ala Arg Leu Glu Val Glu Met Pro
   225                 230                 235                 240
   Gly Glu Thr Asn Leu Leu Cys Thr Leu Arg Glu Tyr Leu Lys His Asn
                   245                 250                 255
   Ile Cys Leu Pro Val Val Glu Ala Val Gln Gly Thr Lys Met Gln Gln
               260                 265                 270
   Phe Gln Ala Pro His Asp Thr Phe Thr Gly Gln Pro Pro Ser Leu Glu
           275                 280                 285
   Met His Trp Ser Ser Phe Gln Ile Asn Ile Asp Pro Ser Ser Phe Asp
       290                 295                 300
   Glu Lys His Ala Ala Ala Val Pro Val Ile Pro Thr Glu Ser Glu Phe
   305 310 315 320
   Gly Cys Thr His Gly Asp Ile Asp Lys Val Glu Gly Ile Leu Thr Thr
   325 330 335
   Ala Pro Arg Ser Phe Asn Ser Glu Asn Thr Cys Leu Ile Val Asp Glu
   340 345 350
   Asp Ile Pro Asp Ser Glu Phe His Gln Met Glu Val Val Glu Glu Val
   355 360 365
   Ser Val Asp Ser Val Pro Asp Gly Glu Lys Gln Asp Glu Met His Gly
   370 375 380
   Ser Ser Ser Pro Thr Asn Leu Asp Pro Ala Phe Leu Asp Glu Lys His
   385 390 395 400
   Val Ala Ala Val Ala Ile Ile Pro Thr Glu Ser Glu Phe Gly Cys Thr
   405 410 415
   Tyr Gly Asp Asn Asp Lys Val Glu Asp Ile Leu Thr Thr Gly Ser Arg
   420 425 430
   Thr Phe Asn Ser Glu Asn Thr Cys Leu Ile Val Asp Lys Asp Ile Pro
   435 440 445
   Asp Ser Glu Phe His Gln Met Glu Val Val Glu Glu Ile Ser Val Asp
   450 455 460
   Ser Val Pro Asp Glu Glu Lys Gln Asp Glu Cys Asp Glu Glu Asn Leu
   465 470 475 480
   Asn Gly Lys Ser Cys Arg Glu Glu Gly Tyr Ser Leu Asp Glu Val Val
   485 490 495
   Glu Asp Asn Gly Asn Lys Cys Ile Lys Asn Ile Asp Pro Ala Ser Phe
   500 505 510
   Asp Gly Lys Gly Leu Thr Ala Val Thr Val Ile Pro Thr Glu Phe Glu
   515 520 525
   Phe Gly Cys Thr Leu Gly Asp Asn Glu Arg Ile Glu Asp Ile Leu Glu
   530 535 540
   Met Glu Ser Arg Thr Ile Asn Ser Glu Asn Thr Ser Leu Leu Asp Glu
   545 550 555 560
   Lys Ala Ile Ala Val Thr Lys Phe Gln Leu Val Asn Ile Val Glu Glu
   565 570 575
   Gly Gly Tyr Ser Leu Asp Glu Val Val Glu Asp Asn Gly Asn Lys Cys
   580 585 590
   Ile Lys Asn Ile Asp Pro Ala Ser Phe Asp Gly Lys Gly Leu Ala Ala
   595 600 605
   Val Thr Val Ile Pro Ser Glu Ser Glu Phe Gly Cys Thr Leu Gly Asp
   610 615 620
   Asn Glu Arg Ile Glu Asp Ile Leu Glu Met Glu Ser Arg Thr Ile Asn
   625 630 635 640
   Ser Glu Asn Thr Ser Leu Leu Asp Glu Asn Ala Ile Ala Val Thr Lys
   645 650 655
   Phe Gln Val Val Asn Ile Val Glu Glu Val Ala Met Asp Ser Ile Ser
   660 665 670
   Asp Gly Asn Lys Cys Ile Lys Asn Ile Asp Pro Ala Ser Phe Asp Gly
   675 680 685
   Lys Gly Leu Ala Ala Val Thr Val Ile Pro Ser Xaa Asp Lys Cys Gly
   690 695 700
   Lys Glu Leu Glu Ser Lys Leu Pro Ala Ser Leu Asn Ala Lys Ser Cys
   705 710 715 720
   His Glu Gln Gly Lys Ser Val Ala Glu Ile Ala Glu Asp Thr Gly Lys
   725 730 735
   Lys Cys Ala Ser Ser Ile Ser Ser Thr Ser Phe Gln Val Glu Ser Pro
   740 745 750
   Asn Ser Ser Met Pro Arg Glu Gln Thr Pro Gln Ser Leu Thr Ala Val
   755 760 765
   Thr Arg Cys Ser Gly Gly Glu Phe Leu Glu Asn His Val Lys Pro Thr
   770 775 780
   Glu Lys Ser Ser Ala Phe Gly Ser Ile Trp Ser Arg Cys Lys Pro Ala
   785 790 795 800
   Ser Ala Pro Leu Val Gln Ala Arg Lys Ser Arg Phe Met Ser Thr Ala
   805 810 815
   Lys Val Gly Thr Glu Val Lys Arg Ser Asn Glu Lys Asn Val Val Ile
   820 825 830
   Asn Lys Leu Met Pro Lys Asp Leu Ser Ala Val Phe Asp Glu Glu Lys
   835 840 845
   Glu Ala Phe Ile Leu Asn Lys Glu Asn Leu Ser Pro Asn Thr Tyr His
   850 855 860
   Leu Gln Phe Met Arg Lys Lys Asp Lys Pro Glu Glu Ile Lys His Ser
   865 870 875 880
   Ile Ser Gln Arg Ser Pro Asn Leu Ser Tyr Phe Ser Pro Arg Ile Tyr
   885 890 895
   Leu Asp Lys Arg Ile Ser Ser Lys Arg Ser Trp Asp Met Val Val Asp
   900 905 910
   Thr Ala Ser Leu Leu Asn Lys Glu Ser Arg Lys Ala Leu Gln Leu Leu
   915 920 925
   Gln Gly Leu Lys Gly Thr Arg Leu Ile Ile Pro Ser Leu Val Ile Arg
   930 935 940
   Glu Leu Gly Ser Met Lys Gln Lys Phe Arg Ile Phe Arg Thr Thr Ser
   945 950 955 960
   Glu Ala Ser Leu Ala Leu Glu Trp Ile Glu Glu Cys Leu Glu Lys Thr
   965 970 975
   Arg Trp Trp Ile His Ile Gln Ser Ser Met Glu Glu Phe Arg Leu Thr
   980 985 990
   Ala Leu Thr His His Ala Ser Pro Gln Thr Arg Phe Ile Glu Glu Ser
   995 1000 1005
   Trp Ala Phe Pro Gly Leu Asn Thr Leu Lys Lys Cys Ala Ser Pro
   1010 1015 1020
   Lys Val Glu Asp His Ile Leu Asp Ser Ala Leu Gln Tyr Gly Arg
   1025 1030 1035
   Lys Glu Asn Val Gly Gln Leu Val Leu Leu Ser Ser Asp Val Ser
   1040 1045 1050
   Leu Lys Ile Lys Ser Met Ala Lys Gly Leu Leu Cys Glu Thr Val
   1055 1060 1065
   Gln Gln Phe Arg Gln Ser Leu Val Asn Pro Phe Ser Glu Arg Phe
   1070 1075 1080
   Met Trp Pro Lys Ser Ser Pro Arg Gly Leu Thr Trp Ser Cys Gln
   1085 1090 1095
   Asp Asp Leu Val Leu Arg Glu Lys Tyr Cys Gly Leu Pro Ser Lys
   1100 1105 1110
   Ala Gly Leu Lys Leu Ile Thr Phe His Asp
   1115 1120
<210> 6
   <211> 1198
   <212> PRT
   <213> Glycine max
<400> 6
   Met Gly Leu Glu Asn Gln Ser Lys Ala Glu Glu Glu Lys Glu Gly Glu
   1 5 10 15
   Ile Pro Val Leu Thr Val Leu Lys Asn Asn Thr Val Leu Lys Asn Ile
   20 25 30
   Phe Ile Val Asn Lys Pro Thr Asp Gln Lys Gln Ser Ser Ala Asp His
   35 40 45
   Val Asn Val Leu Leu Val Gly Arg His Pro Asp Cys Asp Leu Met Leu
   50 55 60
   Thr His Pro Ser Ile Ser Arg Phe His Leu Gln Ile Arg Ser Asn Pro
   65 70 75 80
   Ser Ser Arg Thr Phe Ser Leu Leu Asp Leu Ser Ser Val His Gly Thr
   85 90 95
   Trp Val Ser Gly Arg Arg Ile Glu Pro Met Val Ser Val Glu Met Lys
   100 105 110
   Glu Gly Glu Thr Leu Arg Val Gly Val Ser Ser Arg Val Tyr Arg Leu
   115 120 125
   His Trp Ile Pro Val Ser Arg Ala Tyr Asp Leu Glu Asn Pro Phe Val
   130 135 140
   Ala Gln Leu Asp Ser Val Ala Glu Glu Glu Glu Glu Glu Lys Glu Glu
   145 150 155 160
   Glu Glu Glu Met Gln Asn Leu Ser Cys Cys Pro Ala Glu Met Glu Glu
   165 170 175
   Ile Glu Ser Met Asp Ser Ile Val Glu Asp Ile Ser Ser Leu Phe Leu
   180 185 190
   Asp Glu Asn Val Glu Leu Thr Val Lys Glu Glu Ile Pro Leu Glu Pro
   195 200 205
   Trp Met Leu Glu Asp Met Ile Ser Leu Cys Cys Glu Glu Glu Arg Lys
   210 215 220
   Ser Pro Ser Lys Glu Glu Ala Ile Glu Ile Pro Ser Asp Pro Phe Gly
   225 230 235 240
   Thr Glu Thr Ser Tyr Leu Pro Thr Ile Ser Asp Gly Glu Asn Asn Leu
   245 250 255
   Cys Asp Ser Val Ser Gln Val Leu Ser Pro Thr Tyr Val Glu Ser Leu
   260 265 270
   Val Glu Cys Asp Asp Thr Leu Thr Glu Asn Leu Ser Asp Thr Ser Cys
   275 280 285
   Leu Pro Ala Val Glu Ala Val Leu Glu Thr Lys Met Leu Gln Phe His
   290 295 300
   Thr Pro Pro Asp Ile Phe Thr Ser Pro Leu Pro Ser Gly His Glu Asn
   305 310 315 320
   Leu Phe Glu Lys His Tyr Ser Ser Leu Pro Val Asn Thr Ala Pro Ser
   325 330 335
   Ser Leu Gly Glu Lys Ser Ala Pro Glu Ala Val Ile Met Pro Glu Glu
   340 345 350
   Thr Glu Cys Glu Ser Glu Asp Asp Glu Ser Ile Ile Asp Ile Phe Thr
   355 360 365
   Val Pro Glu Ser Leu His Ala Ala Glu Asp Val Ile Thr Thr Asn Glu
   370 375 380
   Ser Glu Ser Glu Cys Thr Leu Arg Asp Asp Gly Ser Val Thr Asp Ala
   385 390 395 400
   Phe Ile Ala Gly Ala Gly Asn Phe Asn Ser Glu Asp Val Phe Leu Pro
   405 410 415
   Val Glu Glu Val Met Pro Gly Thr Lys Val Glu Gln Ile Lys Ile Val
   420 425 430
   Glu Lys Val Ala Met Asp Ser Leu Ser Asp Glu Gly Lys Gln His Met
   435 440 445
   Tyr Arg Ser Leu Ser Gln Leu Leu Asn Asp Lys Phe Cys His Asp Gln
   450 455 460
   Trp His Ser Leu Asn Glu Ile Val Gln Asp Val Arg Asn Lys His Ala
   465 470 475 480
   Tyr Ser Ile Ser Pro Thr Pro His Gln Ile Glu Ser Val Asn Leu Ser
   485 490 495
   Met Pro Gln Glu Val Val Leu Asn Ile Met Asn Glu Asp Gln Thr Gln
   500 505 510
   His Ser Asp Met Glu Asn Leu Glu Ser Cys Ile Lys Ala Met Glu Lys
   515 520 525
   Thr Ser Thr Asn Ile Trp Ser Arg Arg Gly Lys Ala Thr Ser Ala Pro
   530 535 540
   Gln Val Arg Thr Ser Lys Ser Ile Leu Lys Asn Ala Ala Asn Val Glu
   545 550 555 560
   Val Ala Met Ser Asn Glu Lys Asp Ile Arg Asn Arg Thr Ile Ser Lys
   565 570 575
   Asn Leu Ser Ser Val Leu Asp Gly Glu Val Glu Glu Asp Asp Glu Glu
   580 585 590
   Ile Tyr Thr Pro Asp Lys Glu Asn Ile Ser Pro Asn Thr Leu His Leu
   595 600 605
   Arg Phe Leu Lys Lys Gly Lys Ile Glu Gly Ile Lys His Ser Lys Ser
   610 615 620
   Gln Arg Ser Arg His Ile Leu Arg Asp Thr Phe Asn Cys Asp Ile Tyr
   625 630 635 640
   Pro Asn Glu Ser Ile Asp Pro Thr Leu Cys Asn Met Asn Lys Lys Asp
   645 650 655
   Leu Phe Ser Val Leu Asp Gly Glu Val Lys Glu Lys Glu Ile Phe Ile
   660 665 670
   Pro Glu Glu Glu Asn Leu Asn Pro Asn Ala Leu Gln Leu Arg Leu Leu
   675 680 685
   Lys Lys Lys Gly Lys Val Glu Glu Ile Lys Arg Ser Lys Ser Arg Arg
   690 695 700
   Ser Pro Leu Ser Lys Gly Thr Phe Asn Pro Asp Met Tyr Pro Asn Glu
   705 710 715 720
   Asn Ile Gly Ser Thr Leu Cys Asn Ile Asn Gln Lys Asp Ser Ile Asn
   725 730 735
   Arg Thr Ile Ser Arg Asp Leu Phe Ser Asp Leu Glu Gly Glu Glu Glu
   740 745 750
   Glu Glu Ile Phe Thr Pro Asp Lys Glu Asn Phe Ser Pro Asn Thr Leu
   755 760 765
   His Leu Arg Leu Leu Lys Lys Lys Gly Lys Val Glu Glu Ile Lys His
   770 775 780
   Ser Lys Ser Gln Arg Ser Pro Leu Ser Lys Gly Thr Phe Asn Pro Asp
   785 790 795 800
   Met Tyr Pro Asn Glu Ser Ile Gly Pro Ser Leu Arg Arg Met Asn Gln
   805 810 815
   Lys Asp Val Ile Asn Lys Thr Ile Ser Lys Asp Leu Leu Ser Asp Leu
   820 825 830
   Asp Gly Glu Glu Glu Glu Glu Glu Ile Phe Thr Pro Asp Lys Glu Asn
   835 840 845
   Phe Ser Pro Asn Thr Leu Arg Leu Gln Leu Leu Lys Lys Lys Asp Asn
       850                 855                 860
   Phe Cys Pro Asn Leu Tyr Pro Asp Glu Asn Ile Ile Pro Thr Ser Asn
   865                 870                 875                 880
   Glu Glu Asn Gln Thr Leu Lys Gly Val Gln Asp Gln Lys Leu Gln Arg
                   885                 890                 895
   Asn Pro Phe Ser Ser His Ile Lys Phe Ala Gln Glu Gln Asp Leu Lys
               900                 905                 910
   Asp Arg Val Glu Arg Ile Pro Phe Gln Ser Leu Arg Asn Ser Gly Asp
           915                 920                 925
   Lys Arg Arg Ser Gly Thr Cys Cys Pro Val Ser Ala Ser Lys Ser Leu
       930                 935                 940
   His Phe Ser Asn Cys Gly Gln Ile Leu Asp Gln Arg Phe Asn Pro Ser
   945                 950                 955                 960
   Asp Ile Ser Gly Val Pro Lys Lys Arg Ser Trp Asp Met Ile Val Asp
                   965                 970                 975
   Thr Thr Ser Leu Val Asn Lys Glu Ser Arg Lys Ala Leu Gln Leu Leu
               980                 985                 990
   Gln Gly Leu Lys Gly Thr Arg Leu Ile Ile Pro Arg Leu Val Ile Arg
           995                 1000                 1005
   Glu Leu Asp Arg Met Lys Gln Gln Phe Thr Ile Phe Arg Arg Ile
       1010                 1015                 1020
   Ser Glu Ser Ser Leu Ala Leu Glu Trp Ile Glu Glu Cys Met Val
       1025                 1030                 1035
   Lys Ser Asn Trp Trp Ile His Ile Gln Ser Ser Val Asp Glu Gly
       1040                 1045                 1050
   Arg Leu Ile Ala Pro Thr Pro Pro Ala Ser Pro Leu Thr Gln Phe
       1055                 1060                 1065
   Ser Glu Glu Ser Trp Thr Ser Leu Ser Thr Gln Lys Phe Ser Met
       1070                 1075                 1080
   Glu Ile Ala Ser Pro Thr Val Glu Asp His Ile Leu Asp Phe Ala
       1085                 1090                 1095
   Leu Leu Tyr Arg Arg Asn Gln Asn Asp Gly Gln Leu Ile Leu Leu
       1100                 1105                 1110
   Ser Glu Asp Val Thr Leu Lys Ile Lys Cys Met Ala Glu Gly Leu
       1115                 1120                 1125
   Leu Cys Glu Pro Val Gln Glu Phe Arg Glu Ser Leu Val Asn Pro
       1130                 1135                 1140
   Phe Ser Glu Arg Phe Leu Trp Asp Lys Ser Ile Pro Arg Gly Gln
       1145                 1150                 1155
   Thr Trp Ser Cys Gln Asp Asp Val Val Leu Arg Glu Lys Phe Cys
       1160                 1165                 1170
   Arg Leu Arg Lys Pro Ser Lys Gly Val Ala Ser Gly Leu Lys Leu
   1175 1180 1185
   Ile Leu Leu His Asn Ser Gln Tyr Gly Leu
   1190 1195
<210> 7
   <211> 1092
   <212> PRT
   <213> Sorghum bicolor
<400> 7
   Met Ala Ala Ala Ala Ala Asp Gly Asp Ala Pro Ile Ala Ala Phe Ala
   1               5                   10                  15
   Val Ala Lys Gly Gly Val Val Leu Lys His Ile Phe Leu Asn Ala Pro
               20                  25                  30
   Pro Pro Glu Ala Ala Thr Thr Arg Gly Arg Gly Ala Glu Asp Ser Glu
           35                  40                  45
   Asp Glu Glu Glu Asp Pro Pro Val Met Val Gly Arg His Pro Asp Cys
       50                  55                  60
   His Val Leu Val Asp His Pro Ser Val Ser Arg Phe His Leu Glu Leu
   65                  70                  75                  80
   Arg Cys Arg Arg Arg Gln Arg Leu Ile Thr Val Thr Asp Leu Cys Ser
                   85                  90                  95
   Val His Gly Thr Trp Val Ser Gly Arg Arg Ile Pro Pro Asn Thr Pro
               100                 105                 110
   Val Asp Leu Ala Thr Gly Asp Thr Leu Arg Leu Gly Ala Ser Lys Arg
           115                 120                 125
   Glu Tyr Arg Leu Leu Trp Leu Ser Leu Arg Glu Ala Phe Glu Met Asp
       130                 135                 140
   Asp Leu Met Tyr Met Pro Ser Leu Pro Glu Glu Asp Lys Glu Glu Arg
   145                 150                 155                 160
   Glu Pro His Ala Tyr Lys Glu Pro Asn Ser Gln Leu Val Pro Gly His
                   165                 170                 175
   Arg Asp Ser Val Gly Met Glu Thr His Gln Asp Thr Ser Glu Gln Ile
               180                 185                 190
   Val Ser Glu Asp Ile Thr Phe Pro Ala Lys Val Ala Pro Ser Ala Pro
           195                 200                 205
   Pro Leu Ser Asp Phe Val His Pro Phe Leu Ala Glu Glu Pro Ser Leu
       210                 215                 220
   Ser Gln Phe His Glu Lys Ile Asp Gly Val Thr Glu Glu Lys Leu Val
   225                 230                 235                 240
   Glu Lys Asn Gln Phe Ser Glu Ser Phe Gly Ser Leu Ile Ile Gln Glu
                   245                 250                 255
   Met Pro Gly Thr Leu Thr Asn Ala Gly Lys Ser Ile His Ser Asp Lys
               260                 265                 270
   Lys Asp Ala Ser Asn Lys Met Ser Lys Arg Ser Lys Leu Arg Ser Val
           275                 280                 285
   Lys Ser Leu Cys Val Asp Thr Gly Arg Ser Arg Asp Arg Ser Ser Thr
       290                 295                 300
   Leu Ile His Ser Ile Arg Lys Gly Asp Gln Asn Glu Ile Leu Val Cys
   305                 310                 315                 320
   Ser Gln Ser Cys Gly Thr Glu Cys Thr Ala Cys Ile Ala Leu Phe Gly
                   325                 330                 335
   Ile Ser Glu Val Glu Arg Ala Glu Glu Lys Glu Glu Leu Ile Ala Glu
               340                 345                 350
   Asp Lys Val Asp Met Asn Pro Pro Ala Ser Met Ile Met Glu Gly Thr
           355                 360                 365
   Met Lys Glu Arg Lys Pro Glu Asn Tyr Ile Pro Gln Asp Pro Val Asp
       370                 375                 380
   Ala Lys Leu Gln Lys Lys Val Gly Leu Leu Asp Ser Ala Leu Pro Leu
   385                 390                 395                 400
   His Phe Lys Asp Asp Ala Phe Thr Asp Lys Glu Ile Pro Glu Trp Asn
                   405                 410                 415
   Gly Ala Thr Ile Asp Thr Glu Ser Val Leu Val Ser Glu Asn Leu Ile
               420                 425                 430
   Met Pro Glu Met Lys His Asp Gly Leu Asn His Leu Asn Leu Glu Gly
           435                 440                 445
   Asp Leu Ser Glu Asn Glu Asn Met Asp Pro Asn Asn Val Ala Glu Gly
       450                 455                 460
   Pro Gly Asn Cys Asn Leu Glu Gly Thr Ile Cys Gly Asn Leu Phe Asp
   465                 470                 475                 480
   Asn Leu Asp Thr Glu Gly Ile Glu Glu Asp Glu Glu Ile Cys Pro Met
                   485                 490                 495
   Asp Lys Asp Glu Ile Thr Pro Asn Val Ser Gly Asn Ile Ile Met Glu
               500                 505                 510
   Arg Ser His Ile Gly Leu Lys Pro Thr Ile Ser Gln Gln Leu Met Asp
           515                 520                 525
   Ser Ile Ser Pro Leu Asn Leu Asp His Asp Asp Phe Ser Glu Asn Glu
       530                 535                 540
   Asn Ser Lys Leu Tyr Thr Gly Asp Gln Met Lys Ser Asn Glu Pro Val
   545                 550                 555                 560
   Ser Glu Asn Leu Asn Pro Leu Met Pro Ile Ser His Leu Glu Phe Lys
                   565                 570                 575
   Asp Asp Ile Leu Leu Asp Met Glu Asn Ser Val Pro Ala Leu Glu Lys
               580                 585                 590
   Ser Glu Ala Met Ala Val Arg Gln Glu Asn Leu Phe Ser Glu Lys Glu
           595                 600                 605
   Asn Val Thr Pro Ala Ser Lys Val Lys Thr Asn Val Arg Arg Val Leu
       610                 615                 620
   Gly Thr Arg Met Asp Asn Ser Met Ser Ala Ala Ala Asp Ser Asn Lys
   625 630 635 640
   Lys Lys Val Leu Gly Ser Arg Val Asp Asn Ser Val Ser Thr Glu Asn
   645 650 655
   Ser Ser Asn Lys Lys Gln Cys Ser Glu Leu Ser Ser Lys Ser Glu Lys
   660 665 670
   Phe His Thr Val Asp Phe Asp Val Phe Tyr Ser Asp Lys Glu Asn Leu
   675 680 685
   Thr Pro Ile Ala Ser Gly Gly Met Lys Ala Arg Lys Cys Phe Pro Asn
   690 695 700
   Asp Leu Ser Val Asp Leu Asp Gln Asp Gln Glu Ala Phe Cys Ser Asp
   705 710 715 720
   Lys Glu Asn Leu Thr Pro Leu Ser Ser Ala Ala Arg Lys Thr Arg Asp
   725 730 735
   Met Ser Glu Asn Arg Ala Arg Val Glu Ser Ala Ile Thr Lys Lys Arg
   740 745 750
   Val Ala Asp Arg Leu Pro Phe Gln Thr Leu Leu Ser Asn Ser Pro Leu
   755 760 765
   Arg Pro Ala Ser Ser His Asp Cys Thr Cys Ala Val Ala Gly Pro Thr
   770 775 780
   Asp Ile Thr Ala Gly Asp Leu Val Ile Lys Leu Glu Asp Lys Phe Asn
   785 790 795 800
   Asn Leu Ser Cys Asn Asn Gln Glu Ser Gly Ser Ala Gly Gln Gly Met
   805 810 815
   Lys Thr Trp Thr Met Val Ala Asn Thr Asp Ser Leu Leu Asp Asp Glu
   820 825 830
   Ser Arg Lys Ala Ile Met Leu Leu Lys Gly Leu Lys Gly Thr Arg Leu
   835 840 845
   Phe Ile Pro Arg Ile Val Ile Arg Glu Leu Asp Ser Met Lys Gln Arg
   850 855 860
   Glu Gly Leu Phe Arg Arg Ser Thr Lys Ala Thr Thr Ile Leu Gln Trp
   865 870 875 880
   Ile Glu Glu Cys Met Ala Thr Glu Ser Trp Trp Ile His Val Gln Ser
   885 890 895
   Ser Ala Asp Met Phe Pro Val Ala Ala Pro Thr Pro Pro Ala Thr Pro
   900 905 910
   Ser Ala Gln Arg Ile Asp Glu Glu Met Glu Ile Ser Ser Ser Ser Ser
   915 920 925
   Thr Phe Asn Pro Met Ala Ser Phe Phe Ser Pro Arg Ser Ser Pro Ala
   930 935 940
   Leu Ala Asp Ile Val Ser Pro Arg Pro Glu Asp Arg Val Leu Asp Cys
   945 950 955 960
   Ala Ile Leu Val Ser Arg Leu Arg Arg Ser Gly Glu Lys Val Val Val
   965 970 975
   Leu Ser His Ser Val Ser Leu Lys Ile Lys Ala Met Ala Glu Gly Leu
   980 985 990
   Pro Cys Glu Gly Ala Lys Glu Phe Arg Glu Ser Leu Met Asp Pro Ser
   995 1000 1005
   Ser Arg Arg Phe Met Trp Ala Ala Ser Ala Pro Arg Gly Ala Ala
   1010 1015 1020
   Cys Ser Cys Leu Asp Ala Ser Ala Leu Ala Glu Asn Tyr Tyr Asn
   1025 1030 1035
   Ser His His His Ala Met Lys Arg Ser Gly Val Val Pro Ala Ala
   1040 1045 1050
   Ala Ala Arg Pro Ala Gln Ala Ala Lys Gly Leu Lys Leu Ile Leu
   1055 1060 1065
   Arg His Asn Ser Leu Tyr Ala Gln Ala Thr Thr Glu Thr Pro Pro
   1070 1075 1080
   Leu Leu Ala Ser Ala Leu Ala Ser Val
   1085 1090
<210> 8
   <211> 1096
   <212> PRT
   <213> Zea mays
<220>
   <221> misc_feature
   <222> (1033)..(1033)
   <223> Xaa can be any naturally occurring amino acid
<400> 8
   Met Ala Ala Ala Asp Gly Asp Ala Leu Ile Ala Ala Phe Ala Val Ser
   1               5                   10                  15
   Lys Gly Gly Ile Val Leu Lys His Ile Phe Leu Asn Ala Pro Pro Pro
               20                  25                  30
   Glu Ala Met Cys Gly Ser Gly Gly Arg Glu Val Glu Ser Asp Glu Glu
           35                  40                  45
   Asp Pro Pro Val Met Val Gly Arg His Pro Asp Cys His Val Leu Val
       50                  55                  60
   Asp His Pro Ser Val Ser Arg Phe His Leu Glu Leu Arg Cys Arg Arg
   65                  70                  75                  80
   Arg Gln Ser Leu Ile Thr Val Thr Asp Leu His Ser Val His Gly Thr
                   85                  90                  95
   Trp Val Ser Gly Arg Arg Ile Pro Pro Asn Thr Pro Val Asp Leu Ala
               100                 105                 110
   Thr Gly Asp Thr Leu Arg Leu Gly Ala Ser Lys Arg Glu Tyr Lys Leu
           115                 120                 125
   Leu Trp Leu Ser Leu Arg Glu Ala Phe Glu Met Asp Asp Leu Met Tyr
       130                 135                 140
   Met Pro Ser Leu Pro Glu Glu Asp Lys Glu Glu Pro Tyr Val Lys Glu
   145                 150                 155                 160
   Pro Ser Ser Lys Leu Leu Pro Gly His Arg Asp Ser Val Asn Met Glu
                   165                 170                 175
   Thr His Gln Asp Thr Ser Glu Gln Ile Val Ser Glu Asp Ile Ala Phe
               180                 185                 190
   Pro Ala Lys Val Ala Pro Ser Ala Pro Pro Leu Ser Glu Phe Leu Gln
           195                 200                 205
   Pro Phe Phe Val Glu Glu His Ser Leu Ser Gln Phe His Glu Lys Arg
       210                 215                 220
   Asn Gly Val Thr Glu Glu Lys Leu Val Asp Lys Asn Gln Ile Ser Glu
   225                 230                 235                 240
   Ser Phe Gly Ser Leu Ile Ile Gln Glu Met Pro Gly Thr Leu Thr Asn
                   245                 250                 255
   Ala Gly Lys Ser Ile Gln Ser Gly Glu Gln Glu Asp Ala Ser Asn Lys
               260                 265                 270
   Val Ser Lys Arg Ser Lys Leu Lys Ser Val Lys Ser Leu Arg Val Asp
           275                 280                 285
   Thr Gly Arg Ser Ser Glu Arg Ser Ser Thr Leu Ser His Ser Phe Gln
       290                 295                 300
   Lys Gly Asp Gln Asn Asp Ile Val Val Cys Ser Gln Ser Cys Gly Thr
   305                 310                 315                 320
   Glu Cys Ala Val Cys Ile Ala Leu Phe Gly Ile Ser Glu Ile Glu Lys
                   325                 330                 335
   Ala Glu Glu Lys Glu Glu Leu Ile Ala Glu Asp Asn Val Asp Met Asn
               340                 345                 350
   Pro Pro Ala Ser Met Ile Met Glu Gly Asn Met Asn Glu Arg Lys Pro
           355                 360                 365
   Asp Asn Tyr Ile Pro Gln Asp Pro Ile Gly Ala Lys Leu Gln Lys Lys
       370                 375                 380
   Leu Gly Leu Leu Asp Ser Ala Leu Pro Leu His Phe Lys Asp Asp Val
   385                 390                 395                 400
   Phe Ala Asp Lys Glu Ile Pro Gln Trp Asn Val Ala Ser Val His Thr
                   405                 410                 415
   Glu Ser Glu Leu Leu Ser Glu Tyr Leu Ile Ile Pro Glu Val Lys His
               420                 425                 430
   Asp Asp Leu Asn His Leu Asn Leu Glu Glu Gly Leu Ser Lys Ser Glu
           435                 440                 445
   Asn Ile Asn Pro Asn Lys Ile Thr Glu Gly Pro Gly Asn Cys Gln Leu
       450                 455                 460
   Glu Gly Thr Ile Arg Gly Asn Leu Phe Asp Asn Leu Asp Thr Asp Gly
   465                 470                 475                 480
   Ile Glu Glu Gly Glu Glu Ile Cys Pro Leu Asp Lys Asp Glu Ile Thr
                   485                 490                 495
   Pro Asn Gly Ser Gly Asn Ile Ile Met Glu Arg Ser Asn Ile Val Leu
   500 505 510
   Lys Pro Thr Ile Ser Gln Gln Leu Met Asp Ser Ile Ser Pro Leu Asn
   515 520 525
   Leu Asp His Gly Asp Phe Ser Glu Asn Glu Asn Ser Met Leu Asn Thr
   530 535 540
   Gly Asp Gln Met Lys Leu Asn Glu Pro Val Ser Glu Asn Leu Asn Pro
   545 550 555 560
   Leu Ile Pro Thr Asp Glu Lys Tyr Leu Lys Ser Gln Thr Glu Glu Cys
   565 570 575
   Met Pro Ile Ser Tyr Leu Glu Phe Lys Asp Asp Ile Leu Leu Asp Arg
   580 585 590
   Glu Asn Ser Val Leu Ala Pro Arg Lys Tyr Glu Ala Met Ser Pro Val
   595 600 605
   Arg Gln Glu Asn Leu Phe Ser Asp Lys Glu Asn Val Thr Pro Ala Ser
   610 615 620
   Lys Val Lys Thr Val Val Arg Gly Val Leu Gly Thr Arg Met Asp Asn
   625 630 635 640
   Ser Val Ser Ala Ala Asn Ala Ser Asn Lys Asn Lys Val Leu Gly Ser
   645 650 655
   Arg Val Asp Asn Ser Val Ser Thr Glu Asn Ser Ser Asn Lys Lys Gln
   660 665 670
   Cys Glu Leu Ser Ser Lys Ser Lys Lys Val His Thr Val Asp Phe Asp
   675 680 685
   Val Phe Tyr Ser Asp Lys Glu Asn Leu Thr Pro Ile Ser Ser Gly Gly
   690 695 700
   Met Lys Ala Arg Lys Cys Phe Pro Lys Asp Leu Ser Val Asp Leu Asp
   705 710 715 720
   Gln Asp Gln Glu Ala Phe Cys Ser Asp Lys Glu Asn Leu Thr Pro Leu
   725 730 735
   Ser Ser Ala Ala Arg Lys Thr Arg Asp Met Ser Gly Asn Leu Thr Arg
   740 745 750
   Val Glu Ser Ala Val Thr Lys Lys Arg Val Val Gly Arg Leu Pro Phe
   755 760 765
   Gln Thr Leu Val Ser Asn Ser Pro Leu Arg Pro Ala Ser Ser His Asp
   770 775 780
   Cys Thr Cys Ala Val Ala Arg Pro Ala Gly Val Ala Ala Gly Asp Leu
   785 790 795 800
   Ala Ile Lys Leu Glu Asp Lys Leu Asn Asp Leu Ser Cys Asn Gly His
   805 810 815
   Glu Ser Gly Ser Ala Gly Glu Gly Met Lys Thr Trp Thr Met Val Ala
   820 825 830
   Asn Thr Asp Ser Leu Leu Asp Asp Glu Ser Arg Lys Ala Ile Met Leu
   835 840 845
   Leu Lys Gly Leu Lys Gly Thr Arg Leu Phe Ile Pro Arg Ile Val Ile
   850 855 860
   Arg Glu Leu Asp Ser Met Lys Gln Arg Glu Gly Leu Phe Arg Arg Ser
   865 870 875 880
   Thr Lys Ala Thr Ser Ile Leu Gln Trp Ile Glu Glu Cys Met Ala Arg
   885 890 895
   Glu Ser Trp Trp Ile His Val Gln Ser Ser Ala Asp Met Phe Pro Val
   900 905 910
   Ala Pro Thr Pro Pro Ala Thr Pro Ser Ala Gln Arg Ile Asp Glu Glu
   915 920 925
   Ile Glu Ile Ser Ser Gly Ser Phe Asn Pro Met Met Ala Leu Phe Gly
   930 935 940
   Pro Arg Ser Ser Ala Ala Leu Ala Asp Met Ile Ser Pro Arg Pro Glu
   945 950 955 960
   Asp Arg Val Leu Asp Cys Ala Leu Leu Val Ser Arg Val Arg Ser Asn
   965 970 975
   Glu Lys Val Val Val Leu Ser Asn Ser Val Thr Leu Lys Ile Lys Ala
   980 985 990
   Met Ala Glu Gly Leu Pro Cys Glu Gly Ala Lys Glu Phe Arg Glu Ser
   995 1000 1005
   Leu Val Asp Pro Ser Ser Arg Arg Phe Met Trp Ala Ala Ser Ala
   1010 1015 1020
   Pro Arg Gly Ser Ala Trp Ser Cys Leu Xaa Ala Ser Ala Leu Ala
   1025 1030 1035
   Glu Asn Tyr Tyr Asn Ser Arg His His Ala Met Lys Arg Arg Val
   1040 1045 1050
   Leu Val Ala Ala Arg Pro Ser Glu Ser Glu Ala Ala Lys Gly Leu
   1055 1060 1065
   Lys Leu Ile Leu Arg His Asn Ser Leu Tyr Ala Gln Ala Thr Asp
   1070 1075 1080
   Ala Val Asn Lys Thr Pro Leu Val Ser Leu Ala Ala Val
   1085 1090 1095
<210> 9
   <211> 1226
   <212> PRT
   <213> Medicago truncatula
<400> 9
   Met Ala Ser Ile Val Val Ser Asp Asp Asn Pro Asn Gln Gln Gln Gln
   1               5                   10                  15
   Ser Leu Ile Pro Val Leu Thr Val Phe Lys Asn Asn Ser Ile Leu Lys
               20                  25                  30
   Asn Ile Ile Ile Leu Asn Asn Asn Asn Asn Asn Asn Lys Tyr Asn Asp
           35                  40                  45
   Asp Gln Ile Leu Leu Val Gly Arg His Pro Asn Cys Asn Ile Val Leu
       50                  55                  60
   Phe His Pro Ser Ile Ser Arg Phe His Leu Gln Ile Arg Phe Asn Pro
   65                  70                  75                  80
   Ser Ser Arg Ser Ile Ser Leu Leu Asp Leu Ser Ser Gly Ile Ser Glu
                   85                  90                  95
   Lys Pro Leu Phe Ser Phe Phe Phe Phe Glu Tyr Ser Phe Phe Phe Ile
               100                 105                 110
   Cys Tyr Ser Phe Leu Glu Ser Trp Phe Phe Ile Val Cys Gly Thr Val
           115                 120                 125
   His Gly Thr Trp Val Cys Gly Arg Lys Leu Glu His Gly Val Ser Val
       130                 135                 140
   Asp Leu Lys Glu Gly Asp Thr Phe Gln Leu Gly Ser Ser Ser Arg Val
   145                 150                 155                 160
   Tyr Leu Leu Gln Phe Val Met Leu Ser Leu Val Phe Val Phe Leu Ser
                   165                 170                 175
   Phe Gly Phe Gly Leu Gln Asn Ile Gly Ser Leu Gly Cys Asp Asp Lys
               180                 185                 190
   Arg Lys Asp Gln Ser Asn Asp Glu Thr Phe Glu Asp Glu Asn Asp Ser
           195                 200                 205
   Phe Gly Thr Glu Thr Ser Cys Cys Asn Gly Glu Asn Lys Leu Cys Gly
       210                 215                 220
   Cys His Phe Cys Leu Leu Ser Pro Pro Tyr Thr Gln Ser Val Asp Glu
   225                 230                 235                 240
   Thr Asp Asn Ile Gln Met Gly Glu Ala Cys Pro Glu Val Glu Met Pro
                   245                 250                 255
   Gly Glu Thr Asn Leu Phe Cys Thr Leu Arg Glu Cys Phe Gln Gln Asn
               260                 265                 270
   Ile Cys Ile Pro Val Ala Glu Ala Val Gln Gly Ser Lys Leu His Gln
           275                 280                 285
   Gln Ser Ser Ala Glu Lys Gln Leu Ile Asp Pro Glu Ser Ser Phe Gly
       290                 295                 300
   Glu Lys Gly Asp Gly Ala Val Asp Glu Val Pro Lys Glu Ser Glu Phe
   305                 310                 315                 320
   Glu Gly Thr Phe Glu Tyr Ile Val Thr Thr Gly Gly Arg Val Phe Asn
                   325                 330                 335
   Ser Glu Asp Met Pro Cys Ser Glu Ser His Gln Thr Asn Thr Asn Glu
               340                 345                 350
   Glu Val Ser Val Asp Ser Leu Ser Asp Gly Glu Lys Gln Gly Ser Cys
           355                 360                 365
   Gly Glu Glu Tyr Glu Ser Glu Leu Gln Asn Leu Asn Ala Asn Ser Cys
       370                 375                 380
   His Lys Gln Gln Tyr Ser Pro Asp Glu Ile Val Glu Asp Ile Gly Lys
   385                 390                 395                 400
   Gln Cys Ile Glu Asn Met Asp Pro Ala Ser Ser Glu Glu Asn Gly Val
                   405                 410                 415
   Ala Ala Leu Ser Val Thr Pro Lys Glu His Lys Leu Glu Phe Phe Ser
               420                 425                 430
   Glu Glu Asn Asp Met Ile Asp Asp Val Leu Ser Ser Val Ala Arg Phe
           435                 440                 445
   Phe Asn Ser Glu Asn Thr Ser Ser Leu Val Lys Glu Thr Ile His His
       450                 455                 460
   Val Thr Asn Phe Gln Gln Ile Asn Thr Val Glu Glu Val Ala Ala Val
   465                 470                 475                 480
   Asp Ser Leu Ser Asp Glu Glu Lys Glu Asn Lys Cys Asp Val Glu Phe
                   485                 490                 495
   Lys Ala Tyr Leu Asn Ile Lys Pro Cys Asp Glu Glu Gly Asn Ser Leu
               500                 505                 510
   Val Glu Thr Val Glu Glu Thr Val Lys Ser Phe Gln Thr Glu Ser Val
           515                 520                 525
   Asn Pro Leu Ser Val Asn Thr Tyr Ser Leu Val Glu Asp Ser Ile Pro
       530                 535                 540
   Val Thr Asn Phe Gln Leu Ile Asn Ile Val Asp Glu Val Ala Thr Val
   545                 550                 555                 560
   Asp Ser Leu Ser Asp Glu Glu Lys Glu Asn Glu Cys Asp Glu Glu Phe
                   565                 570                 575
   Lys Ala Tyr Leu Tyr Val Lys Pro Cys Asp Glu Gly Ser Ser Leu Asp
               580                 585                 590
   Glu Thr Val Glu Glu Thr Val Lys Ser Phe Gln Thr Glu Ser Leu Asn
           595                 600                 605
   Pro Ser Ser Gly Asn Thr Ser Ser Leu Val Glu Glu Ala Ile Pro Val
       610                 615                 620
   Thr Asn Phe Gln Leu Ile Asn Ile Val Glu Glu Val Ala Thr Val Asp
   625                 630                 635                 640
   Ser Leu Ser Asn Glu Glu Lys Glu Asn Glu Cys Asp Glu Glu Phe Lys
                   645                 650                 655
   Ala Tyr Leu Tyr Val Lys Pro Cys Asp Glu Glu Gly Asn Ser Leu Asp
               660                 665                 670
   Glu Thr Val Glu Glu Thr Val Lys Ser Phe Gln Thr Glu Ser Leu Asn
           675                 680                 685
   Pro Ser Val Thr Gln Glu Thr Asp Leu Glu Ile Thr Glu Lys Lys Glu
       690                 695                 700
   Asn Gln Thr Leu Gln Ser Leu Val Ala Val Ala Gly Cys Phe Asp Val
   705                 710                 715                 720
   Lys Phe His Glu Asn Cys Val Glu Glu Ser Val Glu Gly Ser Leu Thr
                   725                 730                 735
   Leu Gly Ser Asp Ile Leu Ser Arg Arg Asp Lys Ala Ala Ser Ala Pro
               740                 745                 750
   Gln Asp Arg Thr Arg Lys Ser Arg Leu Leu Asn Thr Pro Asp Val Asp
           755                 760                 765
   Thr Lys Phe Val Met Ser Asn Leu Lys Asp Ile Asn Ile Ile Asn Lys
       770                 775                 780
   Pro Met Pro Gln Asn Ile Phe Ser Asp Leu Asp Glu Glu Glu Met Phe
   785                 790                 795                 800
   Thr Pro Asn Lys Glu Asn Ser Ser Pro Thr Asn Thr Phe His Ser Gln
                   805                 810                 815
   Phe Met Arg Lys Lys Gly Val Leu Glu Glu Ser Lys Ser Ser Lys Ser
               820                 825                 830
   Gln Arg Ala His Asn Leu Lys Ala Ser Phe Ser Ser Ile Ile Tyr Ser
           835                 840                 845
   Ala Glu Arg Cys Thr Ser Ala Ile Ser Asn Lys Glu Asn Gln Thr Pro
       850                 855                 860
   Lys Ser Gln Arg Ala His Asn Leu Lys Ala Ser Phe Ser Pro Ile Ile
   865                 870                 875                 880
   Tyr Ser Ala Glu Arg Ser Thr Ser Ala Ile Ser Asn Lys Glu Asn Leu
                   885                 890                 895
   Thr Pro Arg Glu Ala Arg Glu Trp Lys Ser Gln Arg Ser His Asn Leu
               900                 905                 910
   Arg Ala Ser Phe Ser Pro Ile Ile Tyr Ser Ala Glu Arg Ser Ala Ser
           915                 920                 925
   Ala Ile Ser Asn Lys Glu Asn Leu Thr Pro Lys Glu Ala Leu Glu Trp
       930                 935                 940
   Met Ser Gly Arg Asn Pro Leu Glu Cys Arg Asn Thr Met Glu Leu Arg
   945                 950                 955                 960
   Lys Lys Arg Val Glu Arg Met Pro Leu Gln Ser Leu Ile Ser Ser Gly
                   965                 970                 975
   Gly Asn His Asn Ser Asn Ser Ser Pro Phe Ser Ser Ser Pro Phe Ser
               980                 985                 990
   Ala Ala Lys Ser Ile Leu Gly Val  Thr Val Arg Ser Ser  Asn Cys Gly
           995                 1000                1005
   His Ile  Ser Asp Lys His Ala  Gln Pro Ser Val Arg  Tyr His Lys
       1010                1015                1020
   Ala Glu  Arg Lys Arg Ser Trp  Asp Leu Val Val Asp  Thr Ser Ser
       1025                1030                1035
   Leu Leu  Asn Lys Glu Ser Arg  Lys Ala Leu Gln Leu  Leu Gln Gly
       1040                1045                1050
   Leu Lys  Arg Thr Arg Leu Ile  Ile Pro Gln Ser Ser  Val Ile Arg
       1055                1060                1065
   Glu Leu Gly Ser Met Lys Gln Gln Ile Gly Ile Phe Arg Arg Ile
   1070 1075 1080
   Ser Glu Ala Ala Leu Ala Leu Glu Trp Ile Glu Glu Cys Ile Gly
   1085 1090 1095
   Lys Thr Lys Trp Trp Ile His Ile Gln Ser Ser Met Glu Asp Glu
   1100 1105 1110
   Phe Arg Leu Ile Ala Pro Thr Pro Pro Thr Gln Phe Asn Glu Asp
   1115 1120 1125
   Val Leu Asp Cys Ala Leu Gln Tyr Arg Arg Lys Asp Asn Val Gly
   1130 1135 1140
   Gln Ile Val Leu Leu Ser Asp Asp Val Asn Leu Lys Ile Lys Ser
   1145 1150 1155
   Met Ala Lys Lys Lys Leu Leu Gln Gly Leu Leu Ser Glu Thr Val
   1160 1165 1170
   Gln Gln Phe Arg Gln Ser Leu Val Asn Pro Phe Ser Glu Arg Phe
   1175 1180 1185
   Met Trp Ala Asn Ser Ser Pro Arg Gly Leu Thr Trp Ser Cys Arg
   1190 1195 1200
   Asp Asp Val Val Leu Arg Glu Lys Tyr Cys Cys Leu Pro Ser Lys
   1205 1210 1215
   Ala Gly Leu Lys Leu Leu Ala Thr
   1220 1225
<210> 10
   <211> 878
   <212> PRT
   <213> Solanum lycopersicum
<400> 10
   Met Ala Asp Lys Leu Glu Ile Ser Ser Ile Glu Glu Asp Lys Lys Ile
   1               5                   10                  15
   Pro Val Phe Thr Val Leu Lys Asn Gly Ala Ile Leu Lys Asn Ile Phe
               20                  25                  30
   Leu Leu Asp Asn Pro Pro Pro Cys Ser Asn Gln Glu Ser Glu Ile Glu
           35                  40                  45
   Glu Ile Leu Val Val Gly Arg His Pro Asp Cys Asn Ile Thr Leu Glu
       50                  55                  60
   His Pro Ser Ile Ser Arg Phe His Leu Arg Ile His Ser Lys Pro Ser
   65                  70                  75                  80
   Ser Leu Ser Leu Ser Val Thr Asp Leu Ser Ser Glu Ser Ser Tyr Met
                   85                  90                  95
   Val Leu Asp His His Leu Met Ile Trp Gly Ser Lys Ser Ser Asp Leu
               100                 105                 110
   Leu Ile Cys Ser Gly Lys Val Pro Lys Pro Thr Cys Gly Ile Ser Leu
           115                 120                 125
   Val His Gly Thr Trp Ile Ser Gly Lys Lys Leu Glu Ser Gly Val Lys
       130                 135                 140
   Val Glu Leu Lys Glu Gly Asp Arg Met Gln Leu Gly Gly Ser Ser Arg
   145                 150                 155                 160
   Val Tyr Arg Leu His Trp Val Pro Ile Ser His Ala Tyr Asp Leu Glu
                   165                 170                 175
   Asn Pro Phe Val Pro Thr Leu Cys Glu Ser Glu Pro Glu Glu Ser Thr
               180                 185                 190
   Gln Glu Glu Gln His Gln Asp Glu Ser Gly Phe Ser Leu Gln Asn Asp
           195                 200                 205
   Gln Ile Gln Lys Glu Asp Tyr Asp Met Val Gln Gly Leu Asp Ser Ser
       210                 215                 220
   Phe Ser Gly Met Ser Ser Leu Pro Arg Leu Arg Ser Leu Thr Pro Pro
   225                 230                 235                 240
   Ala Pro Pro Met Leu Asp Lys Asn Val Ala Ala Asn Glu Asn Leu Pro
                   245                 250                 255
   Gly Asn Ile His Glu Glu Gly Glu Ile Ser Leu Arg Gln Pro Ala Tyr
               260                 265                 270
   Gln Ala Asp Lys Glu Asn Ser Ile Pro Glu Ala Leu Leu Val Pro Gly
           275                 280                 285
   Gln Ser Pro Asn Glu Asn Ala Asp Gly Thr Pro Pro Arg Ser Gln Gln
       290                 295                 300
   Arg Cys Ser Ser Ile Trp Ser Arg Arg Gly Lys His Ser Asn Val Gln
   305                 310                 315                 320
   Ile Gln Thr Gly Lys Asp Arg Ala Met Asn Glu Asn Ile Asp Met Glu
                   325                 330                 335
   Thr Glu Val Glu Ser Ile Asn Arg Glu Ile Glu Gly Thr Ile Ser Val
               340                 345                 350
   Ser Lys Asp Leu Phe Ala Ser Gly Asn Lys Asp Lys Glu Glu Glu Val
           355                 360                 365
   Phe Thr Pro Asp Lys Glu Asn His Thr Pro Ser Ser Leu Phe Leu Gly
       370                 375                 380
   Ser Met Lys Lys Ser Cys Leu Ser Glu Met Thr Asn Arg Ser Gly Arg
   385                 390                 395                 400
   Lys Ser Val Leu Ser Asn Met Asp Glu Thr Asp Glu Glu Thr Phe Thr
                   405                 410                 415
   Pro Asp Lys Glu Asn Met Thr Pro Glu Thr Arg Arg Leu Arg Leu Met
               420                 425                 430
   Lys Lys Ile Gly Ser Gln His Gln Ile Lys His Pro Lys Leu Phe Lys
           435                 440                 445
   Ser Ser Ser Leu Lys Leu Val Val Glu Pro Arg Ser Asn Gln Ala Ala
       450                 455                 460
   Gly Cys Val Ser His Lys Lys Glu Lys Leu Gly Ser Thr Thr Lys Ser
   465                 470                 475                 480
   Thr Gln Pro Asn Val Asp Glu Asn Asp Glu Glu Ile Phe Thr Pro Asp
   485 490 495
   Lys Glu Asn Met Thr Pro Asp Thr Arg Leu Met Arg Ser Met Lys Lys
   500 505 510
   Ile Gly Lys Leu Glu Asp Leu Lys Leu Glu Ser Phe Lys Phe Ser Leu
   515 520 525
   Asp Asn Val Val Asp Pro Ile Phe His Gln Asn Gly Thr Pro Phe Ser
   530 535 540
   Ser Glu Lys Asp Asn Leu Asn Asp Lys Val Leu Glu Glu Gln Lys Ser
   545 550 555 560
   Thr Ile Leu Ala Pro Arg Tyr Pro Ala Arg Leu Glu Val Asn Thr Val
   565 570 575
   Lys Asn Arg Met Asp Arg Val Pro Leu Gln Ser Leu Leu Val Asn Tyr
   580 585 590
   Pro Val Lys Thr Ser Ser Ile Ser Pro Glu Glu Asn Ile Lys Leu Arg
   595 600 605
   Asp Tyr Pro Ile Gln His Pro Glu Thr Met Glu Leu Cys Pro Phe Phe
   610 615 620
   Asn Glu Ser Phe Met Glu Lys Lys Arg Trp Thr Ile Val Val Asp Thr
   625 630 635 640
   Gly Ser Leu Leu Asn Lys Glu Ser Arg Lys Ser Leu Gln Leu Leu Gln
   645 650 655
   Gly Leu Arg Arg Thr Tyr Met Ile Ile Pro Arg Thr Val Ile Arg Glu
   660 665 670
   Leu Asp Cys Met Lys Arg Arg Ala Ser Leu Phe Arg Arg Thr Thr Glu
   675 680 685
   Val Ser Ala Ala Leu Glu Trp Ile Glu Asp Cys Met Ile Asn Ala Lys
   690 695 700
   Ala Trp Ile His Val Gln Ser Cys Ala Glu Glu Thr Arg Ala Val Ala
   705 710 715 720
   Pro Thr Pro Pro Ala Thr Ala Pro Leu Ser Leu Phe Ser Glu Glu Asn
   725 730 735
   Gly Met Phe Pro Val Gly Ser His Gln Phe Ser Pro His Ser Gly Leu
   740 745 750
   Met Asp Phe Ala Ser Pro Thr Ala Glu Asp His Ile Leu Glu Tyr Ala
   755 760 765
   Leu Phe Phe Lys Arg Thr Asn Arg Asn Gly Gln Leu Val Leu Leu Ser
   770 775 780
   Asn Asp Leu Thr Met Lys Ile Lys Ala Met Ala Glu Gly Leu Asn Cys
   785 790 795 800
   Glu Thr Ala Glu Glu Phe Arg Glu Ser Leu Val Asn Pro Phe Ser Glu
   805 810 815
   Arg Phe Leu Trp Lys Asp Ser Ser Pro Arg Gly Arg Thr Trp Ser Cys
   820 825 830
   Glu Asp Asp Phe Val Leu Arg Glu Thr Tyr Tyr His Gly Pro Pro Lys
   835 840 845
   Lys Pro Ser Met Ser Gly Glu Ala Ala Lys Gly Leu Lys Leu Ile Leu
   850 855 860
   Leu His Asn Ser His Phe Arg Cys His Ile Ser Thr Ala Ser
   865 870 875
<210> 11
   <211> 153
   <212> PRT
   <213> Solanum tuberosum
<400> 11
   Phe Glu Glu Ile Leu Val Val Gly Leu His Pro Asp Cys Tyr Ile Val
   1 5 10 15
   Leu Glu His Pro Ser Ile Ser Arg Phe His Leu Thr Ile His Ser Asn
   20 25 30
   Pro Ser Ser His Ser Leu Ser Val Ile Asp Leu Ser Ser Val His Gly
   35 40 45
   Ser Trp Ile Ser Gly Asn Lys Ile Glu Pro Gly Val Arg Val Glu Leu
   50 55 60
   Lys Glu Gly Asp Lys Met Lys Leu Gly Gly Ser Arg Arg Glu Tyr Met
   65 70 75 80
   Leu His Trp Ile Pro Ile Ser Arg Ala Tyr Asp Leu Glu Asn Pro Phe
   85 90 95
   Val Ala Pro Leu Cys Glu Glu Glu Pro Phe Lys Glu Met Asp Glu Lys
   100 105 110
   Glu His His Asp Glu Asn Gly Phe Ala Leu His Asn Glu Gly Asp Asp
   115 120 125
   Leu Val Gln Asp Gln Asp Ser Ser Phe Ser Cys Pro Ser Leu Leu Pro
   130 135 140
   Tyr Ile Lys Cys Pro Thr Pro Ser Ala
   145 150
<210> 12
   <211> 132
   <212> PRT
   <213> Helianthus argophyllus
<400> 12
   Pro Gln Ser Leu Lys Tyr Leu Lys Leu Leu Glu Asp Ile Glu Gly Thr
   1 5 10 15
   Gln Leu Phe Leu Thr Lys Thr Val Val Arg Glu Leu Met Asp Ile Glu
   20 25 30
   Ser Gln Asp Asn Phe Phe Asn Arg Ser Ser Lys Lys Ala Ser Leu Ala
   35 40 45
   Leu Glu Trp Ile Asp Glu Cys Met Met Asn Thr Ser Gln Trp Ile His
   50 55 60
   Met Asp Asp Asp Asp Asp Glu Thr Val Arg Arg Ser Ser Thr Val Leu
   65 70 75 80
   Glu Ile Ala Leu Arg Leu Arg Glu Glu Asp Ser Asp Gln Lys Ile Ile
   85 90 95
   Ile Leu Ser Asp Asn Leu Thr Leu Lys Ile Lys Ala Leu Ala Glu Gly
   100 105 110
   Ile Met Cys Glu Ala Ala Glu Glu Phe His Lys Ser Leu Val Asn Pro
   115 120 125
   Phe Ser Glu Arg
   130
<210> 13
   <211> 189
   <212> **PRT**
   <213> Malus domestica
<400> 13
   Ile Arg Arg Ser Pro Met His Ala Leu Ile Asn Leu Val Gly Glu Gly
   1               5                   10                  15
   Lys Met Ser Trp Thr Met Val Ala Asp Ala Thr Thr Leu Leu Asp Lys
               20                  25                  30
   Glu Ser Arg Lys Ser Leu Gln Phe Leu Gln Gly Leu Lys Gly Thr Gln
           35                  40                  45
   Leu Ile Ile Pro Arg Met Val Ile Arg Glu Leu Asp Cys Leu Lys Gln
       50                  55                  60
   Arg Gly Ser Leu Phe Ile Lys Lys Thr Glu Ala Glu Leu Val Leu Glu
   65                  70                  75                  80
   Trp Ile Lys Asp Cys Met Ile Lys Thr Asn Trp Trp Ile His Val Gln
                   85                  90                  95
   Ser Ser Met Glu Asp Gly Arg Leu Ile Ala Pro Thr Pro Pro Ala Ser
               100                 105                 110
   Pro Gln Ser Leu Phe Asn Glu Lys Ser Trp Gly Phe Pro Ser Arg Thr
           115                 120                 125
   Thr Gly Ser Leu Thr Phe Ser Arg Cys Gly Ser Met Met Asp Leu Val
       130                 135                 140
   Ser Pro Ser Pro Glu Asp His Ile Leu Asp Cys Ala Leu Leu Cys Arg
   145                 150                 155                 160
   Arg Met Lys Arg Asn Tyr Gly Gln Pro Val Leu Leu Ser Thr Asp Val
                   165                 170                 175
   Ala Leu Lys Ile Lys Ala Met Glu Glu Gly Leu Leu Cys
               180                 185
<210> 14
   <211> 200
   <212> PRT
   <213> Triticum_aestivum
<400> 14
   Lys Val Trp Thr Met Leu Ala Asp Thr Asp Cys Leu Leu Asp Asp Glu
   1               5                   10                  15
   Ser Arg Lys Ser Ile Met Leu Leu Lys Gly Ile Lys Gly Thr His Leu
               20                  25                  30
   Ile Ile Pro Arg Ile Val Met Arg Glu Leu Glu Gly Met Lys Gln Arg
           35                  40                  45
   Glu Gly Met Phe Lys Arg Ser Ser Lys Ala Thr Ser Ile Met Gln Trp
       50                  55                  60
   Ile Glu Asp Cys Met Glu Asn Glu Ser Trp Trp Ile His Val Gln Ser
   65                  70                  75                  80
   Ser Ser Glu Met Leu Pro Val Ala Pro Thr Pro Pro Ala Thr Pro Thr
                   85                  90                  95
   Glu Thr Gln Arg Asn Ser Glu Glu Ser Glu Ala Thr Ala Ala Gly Ala
               100                 105                 110
   Phe Asn Ser Met Leu Ala Leu Phe Ser Pro Arg Ser Phe Thr Gly Ile
           115                 120                 125
   Phe Ser Pro Arg Ile Leu Ala Asp Ile Asp Ser Pro Lys Thr Glu Asp
       130                 135                 140
   Arg Val Leu Asp Cys Ala Leu Leu Phe Asn Lys Leu Arg Gly Cys Gly
   145                 150                 155                 160
   Gln Asn Met Val Ile Leu Ser Asn Ser Val Asn Leu Lys Ile Lys Ala
                   165                 170                 175
   Met Ser Glu Gly Leu Leu Cys Glu Gly Ala Lys Glu Phe Arg Glu Thr
               180                 185                 190
   Leu Met Asn Pro Cys Ser Glu Arg
           195                 200
<210> 15
   <211> 19
   <212> DNA
   <213> Artificial
<220>
   <223> PCR primer
<400> 15
   acatctccct tgtcgtaac 19
<210> 16
   <211> 19
   <212> DNA
   <213> Artificial
<220>
   <223> PCR primer
<400> 16
   atctctcaat cgttcgttc 19
<210> 17
   <211> 25
   <212> DNA
   <213> Artificial
<220>
   <223> PCR primer
<400> 17
   ctgataccag acgttgcccg cataa 25
<210> 18
   <211> 20
   <212> DNA
   <213> Artificial
<220>
   <223> PCR primer
<400> 18
   tcggagtcac gaagactatg 20
<210> 19
   <211> 21
   <212> DNA
   <213> Artificial
<220>
   <223> PCR primer
<400> 19
   cagtctcact gattattcct g 21
<210> 20
   <211> 21
   <212> DNA
   <213> Artificial
<220>
   <223> PCR primer
<400> 20
   gctttcttcc cttcctttct c 21
<210> 21
   <211> 21
   <212> DNA
   <213> Artificial
<220>
   <223> PCR primer
<400> 21
   aaggctgata ttctgattca t 21
<210> 22
   <211> 21
   <212> DNA
   <213> Artificial
<220>
   <223> PCR primer
<400> 22
   ctcttgttgg tccgtatctt a 21
<210> 23
   <211> 28
   <212> DNA
   <213> Artificial
<220>
   <223> PCR primer
<400> 23
   aacgtccgca atgtgttatt aagttgtc 28
<210> 24
   <211> 21
   <212> DNA
   <213> Artificial
<220>
   <223> PCR primer
<400> 24
   aatcggtgag tcaggtttca g 21
<210> 25
   <211> 21
   <212> DNA
   <213> Artificial
<220>
   <223> PCR primer
<400> 25
   ccatggatga aagcgattta g 21
<210> 26
   <211> 19
   <212> DNA
   <213> Artificial
<220>
   <223> PCR primer
<400> 26
   tcctttcatc ttaatatgc 19
<210> 27
   <211> 19
   <212> DNA
   <213> Artificial
<220>
   <223> PCR primer
<400> 27
   tctgtccacg aattattta 19
<210> 28
   <211> 16
   <212> DNA
   <213> Artificial
<220>
   <223> PCR primer
<400> 28
   cccatgtctc cgatga 16
<210> 29
   <211> 19
   <212> DNA
   <213> Artificial
<220>
   <223> PCR primer
<400> 29
   ggcgtttaat ttgcattct 19
<210> 30
   <211> 20
   <212> DNA
   <213> Artificial
<220>
   <223> PCR primer
<400> 30
   gttttgggaa gttttgctgg 20
<210> 31
   <211> 24
   <212> DNA
   <213> Artificial
<220>
   <223> PCR primer
<400> 31
   cagtctaaaa gcgagagtat gatg 24
<210> 32
   <211> 21
   <212> DNA
   <213> Artificial
<220>
   <223> PCR primer
<400> 32
   gaggatacct cttgctgatt c 21
<210> 33
   <211> 21
   <212> DNA
   <213> Artificial
<220>
   <223> PCR primer
<400> 33
   cctggcctta ggaacttact c 21
<210> 34
   <211> 22
   <212> DNA
   <213> Artificial
<220>
   <223> PCR primer
<400> 34
   caatttgtta ccagttttgc ag 22
<210> 35
   <211> 24
   <212> DNA
   <213> Artificial
<220>
   <223> PCR primer
<400> 35
   tgagtttggt tttttgttat tagc 24
<210> 36
   <211> 20
   <212> DNA
   <213> Artificial
<220>
   <223> PCR primer
<400> 36
   gccttttcaa cctctacttg 20
<210> 37
   <211> 23
   <212> DNA
   <213> Artificial
<220>
   <223> PCR primer
<400> 37
   atggtgatag atgatgatga tac 23
<210> 38
   <211> 362
   <212> DNA
   <213> Arabidopsis thaliana
<400> 38
   gggaactaaa tgaggtgaag cgcagtcgca gttttctctt tagaagaaga acagagattg     60
   cttcttcagc tctggactgg atcgaagaat gtaaggttaa ttcaaaatgg tggattcaag    120
   tccagagtcc aacagaagaa accaaagcaa ttgcaccaac cccaccagtc actccccagt    180
   caaatggctc atcggcattc cccttttcac ttcactggaa caactatgca ccagagattg    240
   attctccgac atcagaagat caagttctcg aatgtgctct tctttatcga aaccgtaacc    300
   gtgacgaaaa actcgttctt cttagcaacg atgtaactct caagatcaaa gccatggcag    360
   ag                                                                   362
<210> 39
   <211> 21
   <212> DNA
   <213> Artificial Sequence
<220>
   <223> PCR primer
<400> 39
   ccatagtgag agttatggag c 21
<210> 40
   <211> 23
   <212> DNA
   <213> Artificial Sequence
<220>
   <223> PCR primer
<400> 40
   ggcgcctttt caacctctac ttg 23

## Description

The invention relates to plants that produce 2n pollen, and to their use in plant breeding.

Polyploidy is the condition of organisms that have more than two sets of chromosomes. It has played a pervasive role in the evolution, adaptation and speciation of many eukaryotes, including yeasts, insects, amphibians, reptiles, fishes and vertebrates (OTTO, Cell 131, 452-62, 2007).

Polyploidy is particularly prominent in plants; it is estimated that 95% of ferns are polyploids and that almost all angiosperms have experienced at least one round of whole genome duplication during the course of their evolution (CUI, et al. Genome Res 16, 738-49, 2006). In addition, many important crop plants, such as potato, cotton, oilseed rape, alfalfa and wheat are current polyploids, while others, such as maize, soybean, and cabbage, retain the vestiges of ancient polyploid events (GAUT & DOEBLEY, Proc Natl Acad Sci USA, 94, 6809-14, 1997; LYSAK et al., Genome Res 15, 516-25, 2005; SCHLUETER, et al. BMC Genomics, 8, 330, 2007). Even plants with small genomes, such as *Arabidopsis thaliana*, have been impacted by polyploidy (BLANC et al., Plant Cell 12, 1093-101, 2000).

The mechanisms responsible for the formation of polyploids in plants are still poorly understood. However, it is now believed that 2n gametes are the major route for polyploidy formation and that they drive gene flow to occur from the diploid progenitors to the new polyploid species. (BRETAGNOLLE & THOMPSON, New Phytologist 129, 1-22, 1995; RAMSEY & SCHEMSKE, Annual Reviews of Ecology and Systematics 29, 467-50 1, 1998).

2n gametes (also known as unreduced gametes or diplogametes) are gametes having the somatic chromosome number rather than the gametophytic chromosome number. They have been shown to be useful for the genetic improvement of several crops (for review, cf. for instance RAMANNA & JACOBSEN, Euphytica 133, 3-18, 2003).

Given their tremendous importance in evolution and agronomy, 2n gametes have focused considerable attention. (VEILLEUX, Plant Breeding Reviews 3, 252-288, 1985) and BRETAGNOLLE & THOMPSON (1995, cited above) provided exhaustive review of the meiotic aberrations that can generate diplogametes. The best documented and described cytological abnormalities leading to 2n gametes formation include abnormal cytokinesis, the skip of the first or second meiotic division, or abnormal spindle geometry. Co-orientation of 2nd division spindles (parallel spindles or fused spindles) is perhaps the most common of the mechanisms responsible for the formation of 2n spores, and is the main mechanism of 2n pollen formation in potato (CARPUTO et al. Genetics 163, 287-94, 2003). Moreover the mode of formation of 2n gametes has a direct impact on their genetic composition. For example, parallel or fused spindles lead to gametes that are completely heterozygous up to the first crossover on each pair of homologues and that are therefore genetically equivalent to those resulting from the absence of first division (apart from the segregation of recombinant chromatids beyond the first crossover). These gametes are different from those resulting from premature or abnormal cytokinesis that usually contain the two sisters chromatids of every chromosome, and that are therefore genetically equivalent to the gametes resulting from the absence of second meiotic division.

Although environmental factors can affect the frequency of 2n gametes, the ability to produce 2n gametes is heritable and has therefore a strong genetic basis (RAMSEY & SCHEMSKE, 1998, cited above). The genetic determination of 2n pollen production has been studied in detail in several species and usually fit the segregation of a major locus in a background of polygenic variation. In most instances, the capacity to form 2n gametes was found to be controlled by a monogenic recessive allele, while the expression of this phenotype was modulated by several other loci (reviewed in BRETAGNOLLE & THOMPSON, 1995, cited above) and external environment.

So far, none of the genes that contribute to 2n gametes production has been identified and characterized at the molecular level, and this lack of information has hampered a broader use of these gametes in man-assisted breeding programs.

The inventors have now characterized in the model plant *Arabidopsis thaliana,* a gene implicated in the formation of 2n gametes in plants. This gene will be hereinafter designated *AtPS1* (for Arabidopsis thaliana parallel spindles). The inventors have found that inactivation of *AtPS1* generates diploid male spores, giving rise to viable diploid pollen grains and to spontaneous triploid plants in the progeny. The sequence of the *AtPS1* gene of *Arabidopsis thaliana,* is available in the TAIR database under the accession number AT1g34355. This gene encodes a protein of 1477 aa, whose sequence is represented in the enclosed sequence listing as SEQ ID NO: 1. The *AtPS1* gene is conserved in higher plants. A search in the sequence databases allowed to identify orthologs of the AtPS1, for instance in *Populus trichocarpa* (SEQ ID NO:2), *Oryza sativa* (SEQ ID NO:3), *Vitis vinifera* (SEQ ID NO:4), *Glycine max,* (SEQ ID NO:5 and 6) *Sorghum bicolour* (SEQ ID NO:7), *Zea mays* (SEQ ID NO:8), (*Solanum lycopersicum* (SEQ ID NO:9), *Medicago truncatula* (SEQ ID NO:10), *Solanum tuberosum* (partial sequence represented as SEQ ID NO:11), *Helianthus_argophyllus* (partial sequence represented as SEQ ID NO:12), *Malus x domestica* (partial sequence represented as SEQ ID NO:13),. and *Triticum_aestivum* (partial sequence represented as SEQ ID NO:14). These proteins bear two domains, a PINc domain (InterPro: IPR006596) which is predicted to play a role in nucleotide-binding, potentially being found in RNases, and a FHA (ForkHead Associated) domain (InterPro:IPR000253) a phosphopeptide recognition domain found in many regulatory proteins.

The invention thus provides a method for obtaining a plant producing 2n pollen, wherein said method comprises the inhibition in said plant of a protein hereinafter designated as PS1 protein, wherein said protein:
- comprises within its N-terminal region (preferably within its 300 N-terminal amino-acids, more preferably within its 250 N-terminal aminoacids, and still more preferably within its 200 N-terminal aminoacids), a domain having at least 40%, and by order of increasing preference, at least 45, 50, 55, 60, 65, 70, 75, 80, 85, 90, 95 or 98% sequence identity, or at least 60%, and by order of increasing preference, at least, 65, 70, 75, 80, 85, 90, 95 or 98% sequence similarity with the FHA domain of the AtPS1 protein (amino acids 64-132 of SEQ ID NO: 1);
- comprises within its C-terminal region (preferably within its 350 C-terminal amino-acids, more preferably within its 300 C-terminal aminoacids, and still more preferably within its 250 C-terminal aminoacids), a domain having at least 40%, and by order of increasing preference, at least 45, 50, 55, 60, 65, 70, 75, 80, 85, 90, 95 or 98% sequence identity, or at least 60%, and by order of increasing preference, at least, 65, 70, 75, 80, 85, 90, 95 or 98% sequence similarity with the PINc domain of the AtPS1 protein (amino acids 1237-1389 of SEQ ID NO: 1).

Unless otherwise specified, the protein sequence identity and similarity values provided herein are calculated using the BLASTP program under default parameters. Similarity calculations are performed using the scoring matrix BLOSUM62.

By way of non-limitative examples of representative PS1 proteins of various plant families, one can cite, besides the *AtPS1* of SEQ ID NO: 1, the *Populus trichocarpa* PS1 protein of SEQ ID NO: 2, , the *Oryza sativa* PS1 protein of SEQ ID NO: 3, the *Vitis vinifera* PS1 protein of SEQ ID NO:4, the *Glycine max* PS1 proteins of SEQ ID NO:5 and 6, the *Sorghum bicolour* PS1 protein of SEQ ID NO:7, the *Zea mays* PS1 protein of SEQ ID NO:8, the *Solanum lycopersicum* PS1 protein of SEQ ID NO:9, the *Medicago truncatula* PS1 protein of SEQIDNO:10, the *Solanum tuberosum* PS1 protein (partial sequence SEQ ID NO:11), the *Helianthus_argophyllus* PS1 protein (partial sequence SEQ ID NO:12), the *Malus x domestica* PS1 protein (partial sequence SEQ ID NO:13), and the *Triticum_aestivum* PS1 protein (partial sequence as SEQ ID NO:14).

The inhibition of a PS1 protein can be obtained either by abolishing, blocking, or decreasing its function, or advantageously, by preventing or down-regulating the expression of its gene.

By way of example, inhibition of said PS1 protein can be obtained by mutagenesis of the corresponding gene or of its promoter, and selection of the mutants having partially or totally lost the PS1 protein activity. For instance, a mutation within the coding sequence can induce, depending on the nature of the mutation, the expression of an inactive protein, or of a protein with impaired activity; in the same way, a mutation within the promoter sequence can induce a lack of expression of said PS1 protein, or decrease thereof.

Mutagenesis can be performed for instance by targeted deletion of the *PS1* coding sequence or promoter, or of a portion thereof, or by targeted insertion of an exogenous sequence within said coding sequence or said promoter. It can also be performed by inducing random mutations, for instance through EMS mutagenesis or random insertional mutagenesis, followed by screening of the mutants within the *PS1* gene. Methods for high throughput mutagenesis and screening are available in the art. By way of example, one can mention TILLING (Targeting Induced Local Lesions IN Genomes, described by McCallum *et al.,* 2000). Among the progeny of the mutants having a mutation within the *PS1* gene, the plants which are homozygous for the mutation have the ability to produce 2n pollen; these plants can be identified among on the basis of their phenotypic characteristics, for instance the formation of at least 5%, preferably at least 10%, and more preferably at least 20% of dyads as a product of male meiosis.

Advantageously, the inhibition of said PS1 protein is obtained by silencing of the corresponding *PS1* gene. Methods for gene silencing in plants are known in themselves in the art. For instance, one can mention by antisense inhibition or co-suppression, as described by way of example in U.S. Patents 5,190,065 and 5,283,323. It is also possible to use ribozymes targeting the mRNA of said PS1 protein.

Preferred methods are those wherein gene silencing is induced by means of RNA interference (RNAi), using a silencing RNA targeting the *PS1* gene to be silenced. Various methods and DNA constructs for delivery of silencing RNAs are available in the art.

A "silencing RNA" is hereindefined as a small RNA that can silence a target gene in a sequence-specific manner by base pairing to complementary mRNA molecules. Silencing RNAs include in particular small interfering RNAs (siRNAs) and microRNAs (miRNAs).

Initially, DNA constructs for delivering a silencing RNA in a plant included a fragment of 300 bp or more (generally 300-800 bp, although shorter sequences may sometime induce efficient silencing) of the cDNA of the target gene, under transcriptional control of a promoter active in said plant. Currently, the more widely used silencing RNA constructs are those that can produce hairpin RNA (hpRNA) transcripts. In these constructs, the fragment of the target gene is inversely repeated, with generally a spacer region between the repeats (for review, cf. WATSON *et al.,* 2005). One can also use artificial microRNAs (amiRNAs) directed against the *PS1* gene to be silenced (for review about the design and applications of silencing RNAs, including in particular amiRNAs, in plants cf. for instance OSSOWSKI et al., (Plant J., 53, 674-90, 2008).

The present invention provides chimeric DNA constructs for silencing a *PS1* gene, including in particular expression cassettes for hpRNA, or amiRNA targeting the *PS1* gene.

An expression cassette of the invention may comprise for instance:
- a promoter functional in a plant cell;
- a DNA construct of 200 to 1000 bp, preferably of 300 to 900 bp, comprising a fragment of a cDNA encoding a PS1 protein or of its complementary, or having at least 95% identity, and by order of increasing preference, at least 96%, 97%, 98%, or 99 % identity with said fragment, said DNA sequence being placed under transcriptional control of said promoter.
   According to a preferred embodiment of the invention, an expression cassette for a hpRNA
- a promoter functional in a plant cell,
- a DNA construct which is capable, when transcribed, of forming a hairpin RNA targeting the *PS1* gene;
said DNA construct being placed under transcriptional control of said promoter.

Generally, said hairpin DNA construct comprises: i) a first DNA sequence of 200 to 1000 bp, preferably of 300 to 900 bp, consisting of a fragment of a cDNA encoding a PS1 protein, or having at least 95% identity, and by order of increasing preference, at least 96%, 97%, 98%, or 99 % identity with said fragment; ii) a second DNA sequence that is the complementary of said first DNA, said first and second sequences being in opposite orientations and ii) a spacer sequence separating said first and second sequence, such that these first and second DNA sequences are capable, when transcribed, of forming a single double-stranded RNA molecule. The spacer can be a random fragment of DNA. However, preferably, one will use an intron which is spliceable by the target plant cell. Its size is generally 400 to 2000 nucleotides in length.

According to another preferred embodiment of the invention, an expression cassette for a amiRNA targeting the *PS1* gene comprises:
- a promoter functional in a plant cell,
- a DNA construct which is capable, when transcribed, of forming an amiRNA targeting the *PS1* gene;
said construct being placed under transcriptional control of said promoter.

A large choice of promoters suitable for expression of heterologous genes in plants is available in the art.

They can be obtained for instance from plants, plant viruses, or bacteria such as *Agrobacterium*. They include constitutive promoters, *i.e*. promoters which are active in most tissues and cells and under most environmental conditions, as well as tissue-specific or cell-specific promoters which are active only or mainly in certain tissues or certain cell types, and inducible promoters that are activated by physical or chemical stimuli, such as those resulting from nematode infection.

Non-limitative examples of constitutive promoters that are commonly used in plant cells are the cauliflower mosaic virus (CaMV) 35S promoter, the Nos promoter, the rubisco promoter, the Cassava vein Mosaic Virus (CsVMV) promoter.

Organ or tissue specific promoters that can be used in the present invention include in particular promoters able to confer meiosis-associated expression, such as the *DMC1* promoter (KLIMYUK & JONES, Plant J, 11, 1-14, 1997); one can also use the endogenous promoter of *PS1*.

The DNA constructs of the invention generally also include a transcriptional terminator (for instance the 35S transcriptional terminator, or the nopaline synthase (Nos) transcriptional terminator).

The invention also includes recombinant vectors containing a chimeric DNA construct of the invention. Classically, said recombinant vectors also include one or more marker genes, which allow for selection of transformed hosts.

The selection of suitable vectors and the methods for inserting DNA constructs therein are well known to persons of ordinary skill in the art. The choice of the vector depends on the intended host and on the intended method of transformation of said host. A variety of methods for genetic transformation of plant cells or plants are available in the art for many plant species, dicotyledons or monocotyledons. By way of non-limitative examples, one can mention virus mediated transformation, transformation by microinjection, by electroporation, microprojectile mediated transformation, *Agrobacterium* mediated transformation, and the like.

The invention also provides a host cell comprising a recombinant DNA construct of the invention. Said host cell can be a prokaryotic cell, for instance an Agrobacterium cell, or a eukaryotic cell, for instance a plant cell genetically transformed by a DNA construct of the invention. The construct may be transiently expressed; it can also be incorporated in a stable extrachromosomal replicon, or integrated in the chromosome.

The invention also provides a method for producing a transgenic plant able to produce 2n pollen, said method comprising the steps consisting of:
- transforming at least one plant cell with a vector containing a DNA construct of the invention;
- cultivating said transformed plant cell in order to regenerate a plant having in its genome a transgene containing said DNA construct.

The invention also provides plants genetically transformed by a DNA construct of the invention. Preferably, said plants are transgenic plants, wherein said construct is contained in a transgene integrated in the plant genome, so that it is passed onto successive plant generations. The expression of said chimeric DNA constructs, resulting in a down regulation of the PS1 protein, provides to said transgenic plant the ability to produce 2n pollen.

The invention also encompasses a method for producing 2n pollen, wherein said method comprises obtaining a plant producing 2n pollen by a method of the invention, cultivating said plant and recovering the pollen produced by said plant. Preferably said pollen comprises at least 10%, more preferably at least 20%, and by order of increasing preference, at least 30%, 40%, 50%, or 60 % of viable 2n pollen grains.

The 2n pollen produced by the plants of the invention is useful in particular in plant breeding, for producing polyploids plants (for instance sterile triploids), or to allow crosses between plants of different ploidy level.

The present invention applies to a broad range of monocot- or dicotyledon plants of agronomical interest. By way of non-limitative examples, one can mention potato, tomato, alfalfa, sugar cane, sweet potato, manioc, blueberry, clover, soybean, ray-grass, banana, melon, watermelon or ornamental plants such as roses, lilies, tulips, narcissus.

Foregoing and other objects and advantages of the invention will become more apparent from the following detailed description and accompanying drawings. It is to be understood however that this foregoing detailed description is exemplary only and is not restrictive of the invention.

### DESCRIPTION OF THE DRAWINGS:

### Figure 1: AtPS1 gene structure

Intron/exon structure of the *AtPS1* gene and location of the three different T-DNA insertions (triangles). The primers used are indicated below the gene diagram.

### Figure 2: Multiple sequence alignment representing segments of highest sequence conservation among plant AtPS1 proteins

Full-length AtPS1 proteins of *Arabidopsis thaliana* (At: NP_564445) *Populus trichocarpa* (Pt: jgi_592219), *Oryza sativa* (Os:|NP_001065865), *Vitis vinifera* (Vv: CAN81434_mod), *Glycine max* (Gm: jgi_scaffold_143 and jgi_scaffold_131), *Sorghum bicolour* (Sb:jgi_5039668), and *Zea mays* (Zm :EST_10287.m000022) were aligned and segments of highest conservation were identified using plotcon (EMBOSS package).

The sequences shown in this alignment are derived from those represented in the enclosed sequence listing under SEQ ID NO: 1 to 8 by removal of the non-conserved sequence segments from the alignment. The length of these deleted regions is indicated in box brackets in the corresponding position of the alignment. Domain hits based on a comparison against the Interpro domain database (PMID: 18428686) are indicated by gray boxes above the alignment, and the hit-indicators are extended to include adjacent segments of sequence and structure conservation. In addition, a short motif identified as a repeat element C in *Arabidopsis thaliana* AtPS1, is marked using white boxes.

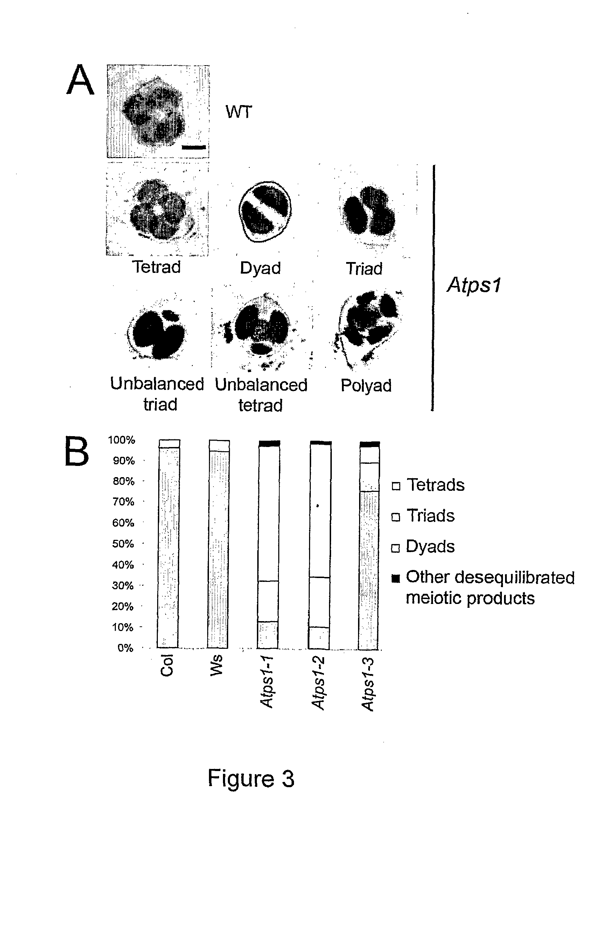

### Figure 3: Wild type and Atps1 mutants meiotic products analysis.

A: Photos of meiotic products in wild type and *Atps1* mutants. Scale bar=10µm. B: Quantification of meiotic products in Ws-4 (n=92), Col-0 (n=212), *Atps1-3* (n=436), *Atps1-1* (n=1125), *Atps1-2* (n=554), the *Atps1-1*/*Atps1-3* F1 (n=283) and *Atps1-1*/*Atps1-2* F1 (n=252).

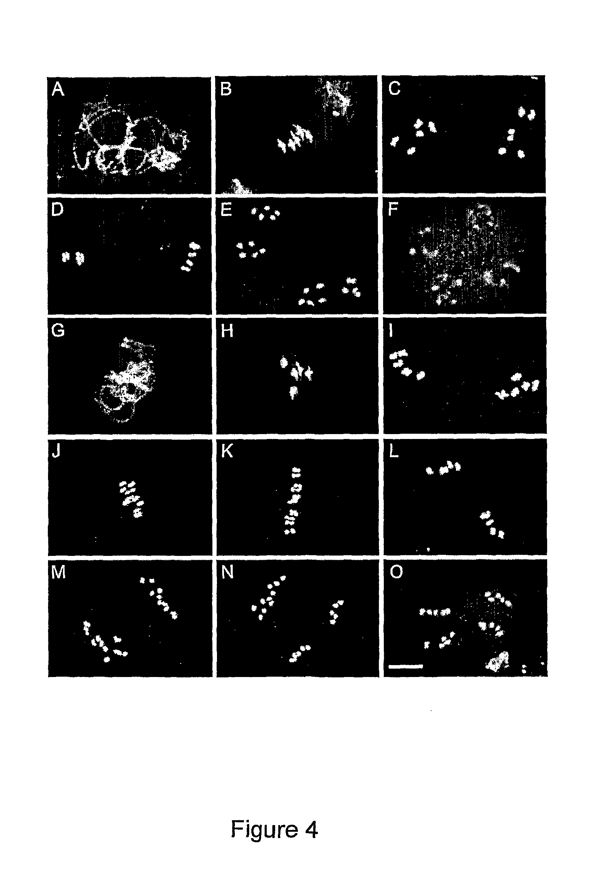

### Figure 4: Meiotic chromosome spreads of wild type and Atps1.

A-F: Wild type meiotic chromosome spreads. A: pachytene. B: metaphase I. C: anaphase I. D: metaphase II. E: Anaphase II F: telophase II. G-O *Atps1-1* meiosis. G: pachytene. H: metaphase II: anaphase I J-L: metaphase II. M-O: Anaphase II. M: dyad. N: triad. O: tetrad. Scale bar=10µm.

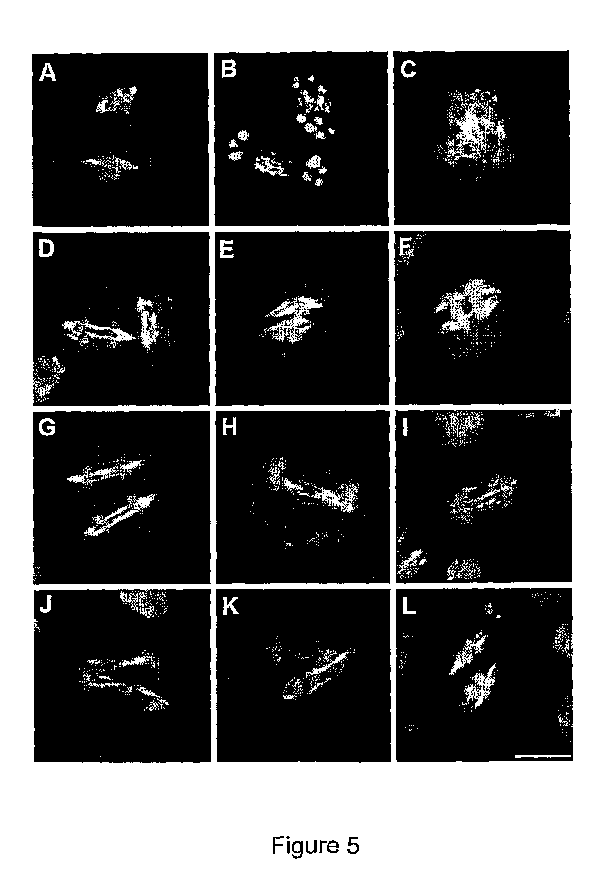

### Figure 5: Immuno-staining of meiosis II spindles in wild type and Atps1-1 mutant.

A, B, C: Wild type spindles at metaphase II, anaphase II and telophase II, respectively. D to L: *Atps1-1* meiocyte at metaphase II / anaphase II. Chromosomes were stained by propidium iodide or DAPI and microtubules by immunolocalisation. Scale bar=5µm.

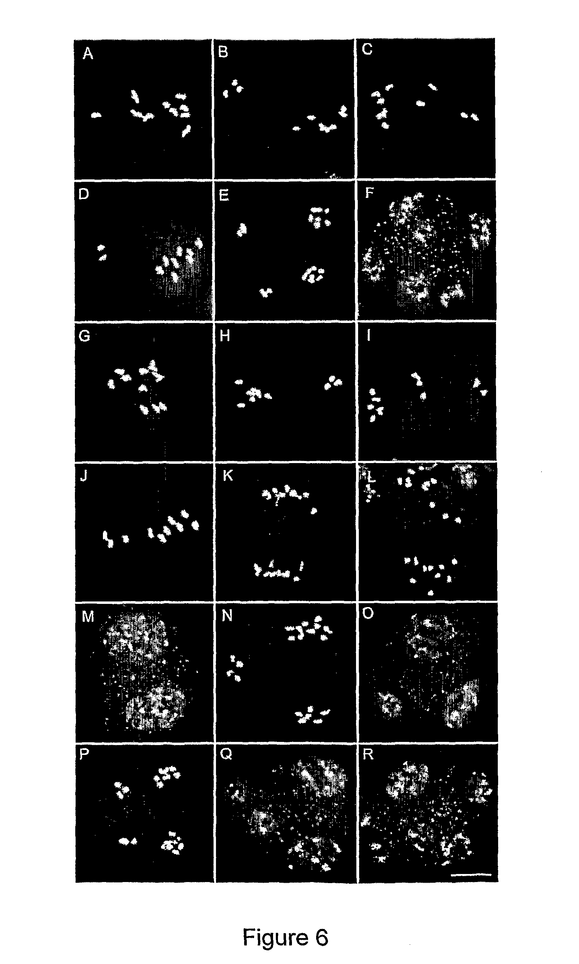

### Figure 6: Meiotic chromosome spreads of Atspo11-1 and Atps1-1/Atspo11-1 double mutant.

A to F: *Atspo11-1* meiotic chromosome spreads. A: metaphase I. B and C: anaphase I. D: metaphase II. E: Unbalanced Tetrad. F: Polyad. G to R: *Atps1-1*/*Atspo11-1* meiosis. G: metaphase I. H and I: anaphase I. J: metaphase II. K: Anaphase II. L and M: Balanced dyad II. N and O: Triad. P and Q: Unbalanced tetrad. R: Polyad. Scale bar=10µm.

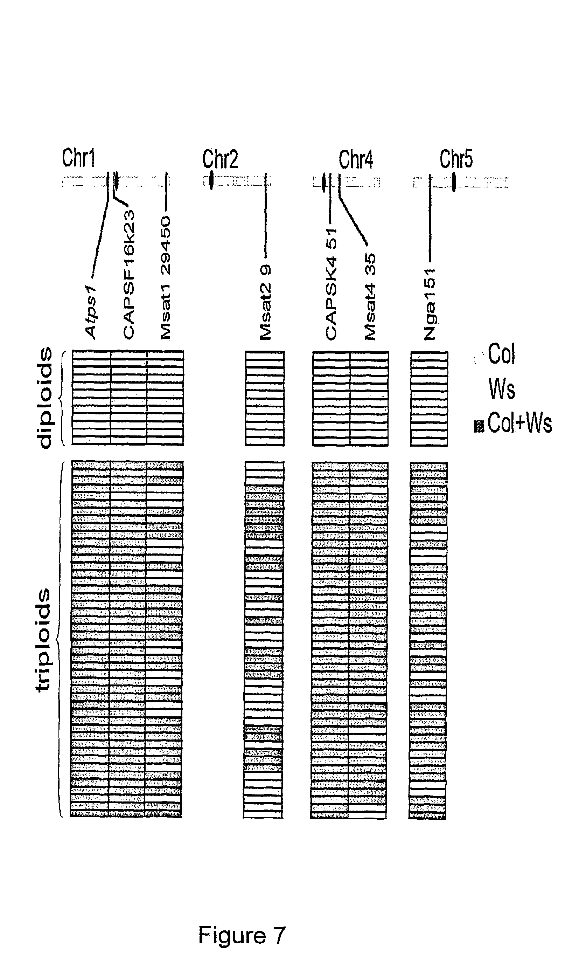

### Figure 7: Genotype of offspring of Atps1 mutants.

Diploid and triploid offspring of the *Atps1-1(Col-0)* / *Atps1-3(Ws-4)* ♂ x Ler ♀ cross was genotyped for several genetic markers. For each marker plants bearing only the Col-0 allele are in medium grey, plants bearing only the Ws-4 allele are in light grey and plants bearing both the Col-0 and Ws-4 alleles are in dark grey. The Ler alleles are present in all the plants because it was used as the female parent in the cross. The position of each marker and the centromeres are indicated along the chromosomes.

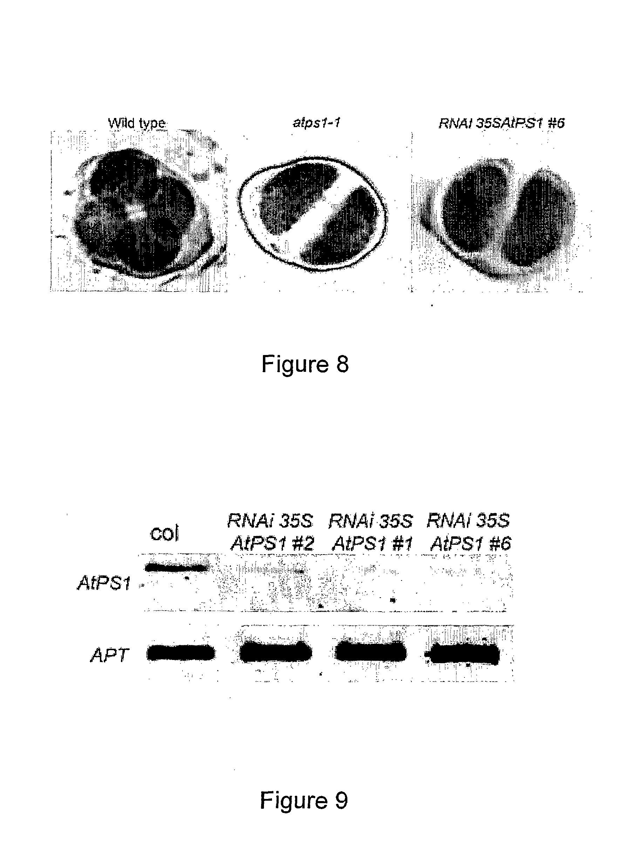

### Figure 8. Meiotic products of wild type, atps1-1 mutant and RNAI35AtPS1#6 plant.

### Figure 9. Expression of AtPS1 in three RNAI35AtPS1 plants.

### EXAMPLES

### EXPERIMENTAL PROCEDURES

### Plant material and growth conditions.

The wild-type references plant material used in this study were *A. thaliana* accession Columbia (Col-0) and Wassilewskija (Ws-4). The T-DNA insertion mutant lines N578818 (ALONSO et al., Science, 301, 653-7, 2003) (*Atps1-1*), N851945 (WOODY et al., J Plant Res 120, 157-65, 2007) (*Atps1-2*), N646172 (*Atspo11-1-3:* STACEY et al. Plant Journal 48, 206-216 2006) obtained from the European Arabidopsis stock center (SCHOLL et al., Plant Physiol. 124, 1477-80, 2000) and EQM96 (*Atps1-3*) from the Versailles T-DNA collection (SAMSON et al., Nucleic Acids Res 30, 94-7, 2002) were used.

*Arabidopsis* plants were cultivated in a greenhouse or growth chamber under the following conditions: photoperiod 16 h day/8 h night; temperature 20 °C day and night; humidity 70%. For germination assay and cytometry experiments *Arabidopsis* were cultivated *in vitro* on *Arabidopsis* medium (ESTELLE et al., Mol. Gen. Genet. 206, 200-206, 1987) at 21 °C with a photoperiod of 16h day/8h night, and 70% of hygrometry.

### Genetic analysis.

Genotyping of T-DNA insertion mutants was done by PCR (30 cycles of 30s at 94°C, 30s at 56°C and 1min at 72°C) using two couple of primers. For each line the first couple designated is specific of the wild type locus and second couple is specific of the T-DNA insertion.
Atps1-3: EQM96L (5'ACATCTCCCTTGTCGTAAC3': SEQIDNO:15) and EQM96U (5'ATCTCTCAATCGTTCGTTC3': SEQ ID NO:16); EQM96L and tag3 (5' CTGATACCAGACGTTGCCCGCATAA3': SEQ ID NO:17). Atps1-1: N578818U2 (5'TCGGAGTCACGAAGACTATG3': SEQ ID NO:18) and N578818L (5'CAGTCTCACTGATTATTCCTG 3': SEQ ID NO:19) ; N578818U2 and LbSalk2 (5' GCTTTCTTCCCTTCCTTTCTC 3': SEQ ID NO:20). Atps1-2: N851945U (5'AAGGCTGATATTCTGATTCAT3': SEQ ID NO:21) and N851945L (5' CTCTTGTTGGTCCGTATCTTA3': SEQ ID NO:22) ; N851945U and P745 (5'AACGTCCGCAATGTGTTATTAAGTTGTC3': SEQ ID NO:23). spo11-3: N646172U (5' AATCGGTGAGTCAGGTTTCAG3': SEQ ID NO:24) and N646172L (5' CCATGGATGAAAGCGATTTAG3': SEQ ID NO:25) ; N646172L/ LbSalk2.

Double spo11/Atps1 mutants were obtained as described in VIGNARD et al. (PLoS Genet, 3, 1894-906,2007)

### Genetic markers used to genotype Atps1-1/Atps1-3 x Ler F1 triploid and diploid plants

Microsatellite msat1.29450 (located on chromosome I at position 29450001) was amplified (Tm=57°C) using 5'TCCTTTCATCTTAATATGC3' (SEQ ID NO:26) and 5'TCTGTCCACGAATTATTTA3' (SEQ ID NO:27) primers. Microsatellite Msat4.35 (Tm=58°C) (located on chromosome 4 at position 7549125) was amplified using 5'CCCATGTCTCCGATGA3' (SEQ ID NO:28) and 5'GGCGTTTAATTTGCATTCT3' (SEQ ID NO:29) primers. Microsatellite NGA151 (Tm=58°C) (located on chromosome 5 at position 4669932) was amplified using 5'GTTTTGGGAAGTTTTGCTGG3' (SEQ ID NO:30) and 5'CAGTCTAAAAGCGAGAGTATGATG3' (SEQ ID NO:31) primers. The 2 primer pairs specific for the *Atps1-1* and *Atps1-3* TDNA borders were used as a centromeric marker of the chromosome 1. CAPS markers Seqf16k23 (physical position: 14481813) and CAPSK4 51 (physical position: 5078201) were used as centromeric markers for chromosome 1 and 4, respectively.

CAPS Seqf16k23 is amplified (Tm=60°C) using 5'GAGGATACCTCTTGCTGATTC3' (SEQ ID NO:32) and 5'CCTGGCCTTAGGAACTTACTC3' (SEQ ID NO:33) primers and observed after TaqI digestion.

CAPS CAPSK4 51 is amplified (Tm=60°C) using 5'CAATTTGTTACCAGTTTTGCAG3' (SEQ ID NO:34) and 5'TGAGTTTGGTTTTTTGTTATTAGC3' (SEQ ID NO:35) primers and observed after Mnll digestion.

PCR conditions: 40 cycles of 20s at 94°C, 20s at Tm and 30s at 72°C.

### RT-PCR

Arabidopsis total RNA were extracted using the QIAGEN RNA kit.

Reverse transcription were done on 5µg of total RNA using an oligo(dT) (ALTSCHUL et al., Nucleic Acids Res 25, 3389-402, 1997) as primer. The RevertAid™ M-MuLV Reverse Transcriptase enzyme (FERMENTAS) was used according to the instructions of the manufacturer. RT-PCR were done on 1µl of cDNA using the pAtpsF 5'GCCTTTTCAACCTCTACTTG3' (SEQ ID NO:36) and pAtpsR 5'ATGGTGATAGATGATGATGATAC3' (SEQ ID NO:37) primers under the following conditions: 30 cycles of 30s at 94°C, 30s at 56°C and 1min at 72°C.

### Cytology and Flow Cytometry:

Final meiotic products were observed as described in AZUMI et al., (Embo J, 21, 3081-95., 2002) and viewed with a conventional light microscope with a 40X dry objective. Chromosomes spreads and observations were carried out using the technique described in MERCIER et al., (Biochimie, 83, 1023-28, 2001). The DNA fluorescence of spermatic pollen nuclei was quantified using open LAB 4.0.4 software. For each nucleus the surrounding background was calculated and subtracted from the global fluorescence of the nucleus. Meiotic spindles were observed according to the protocol described in MERCIER et al., (Genes Dev, 15, 1859-71, 2001) except that the DNA was counter-stained with DAPI. Observations were made using an SP2 leica confocal microscope. Images were acquired with a 63X water objective in xyz and 3D reconstructions were made using leica software. Projections are shown. Cells were imaged at excitation 488nm and 405nm with AlexaFluor488 and DAPI respectively. *Arabidopsis* genome sizes were measured as described in MARIE & BROWN, (Biol Cell, 78, 41-51, 1993) using tomato Lycopersicon esculentum cv "Montfavet" as the standard. (2C=1.99 pg, %GC=40.0%)

### EXAMPLE 1: IDENTIFICATION OF THE ARABIDOPSIS AtPS1 GENE AND atps1 MUTATIONS

As a part of a screen for meiotic mutants, the At1g34355 gene was selected as a potential meiotic gene according to its expression profile.Using the Expression Angler tool (TOUFIGHI et al. Plant J 43, 153-63, 2005) with the AtGenExpress tissue set (SCHMID et al., Nat Genet 37, 501-6, 2005) this gene was found to be co-regulated with genes known to be involved in meiosis (*AtMER3, AtDMC1, SDS, AtMND1, AtHOP2*), with the highest expression level in shoot apex and young flower buds.

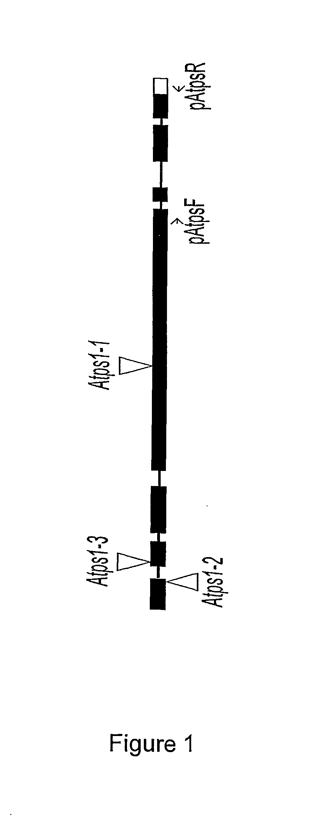

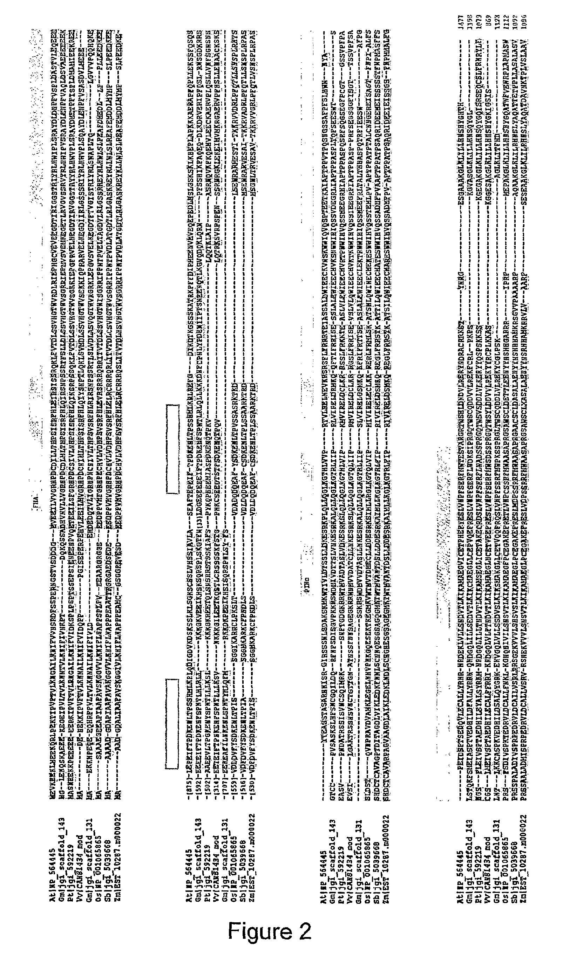

We amplified the *AtPS1* cDNA (EU839993) by RT-PCR on bud cDNA and sequencing confirmed that it is identical to that predicted in the databases (NM_103158). The *AtPS1* gene contains 7 exons and 6 introns (Figure 1) and encodes a protein of 1477 amino acids. BlastP and Psi-Blast (ALTSCHUL et al., Nucleic Acids Res, 25, 3389-402, 1997) analyses showed that the AtPS1 protein is conserved throughout the plant kingdom and contains two highly conserved regions (Figure 2).

An FHA domain (forkhead associated domain) was predicted at the N terminus by CD searches (MARCHLER-BAUER & BRYANT, Nucleic Acids Res, 32, W327-31, 2004), while the C terminal conserved region shows similarity to a PIN Domain. These domains are separed by a compositionally biased sequence of variable length.

An FHA domain is a phosphopeptide recognition motif implicated in protein-protein interactions and is found in a diverse range of proteins involved in numerous processes including intracellular signal transduction, cell cycle control, transcription, DNA repair and protein degradation(DUROCHER & JACKSON, FEBS Lett, 513, 58-66, 2002). The PIN domain was predicted to have RNA-binding properties often associated with Rnase activity(CLISSOLD & PONTING, Curr Biol, 10, R888-90, 2000), and this has now been experimentally confirmed (GLAVAN et al., Embo J, 25, 5117-25, 2006). Accordingly, several PIN domain containing proteins are involved in Rnai, RNA maturation, or RNA decay. We could not identify non-plant proteins with significant similarity to AtPS1 (apart from the FHA and the PIN domains) or which contained both a FHA and a PIN domain.

While a single AtPS1 representative is usually found per species, gene duplication events have occurred in individual lineages such as in *Glycine max.*

We investigated the role of the *AtPS1* gene by isolating and characterising a series of allelic mutants, identified in several public T-DNA insertion line collections(ALONSO et al., Science, 301, 653-7, 2003; SAMSON et al., Nucleic Acids Res, 30, 94-7, 2002; WOODY et al., J Plant Res, 120, 157-65, 2007). The *Atps1-1* (SALK_078818) and *Atps1-2* (WiscDsLox342F09) lines were obtained from the European Arabidopsis stock centre (SCHOLL et al., Plant Physiol, 124, 1477-80, 2000) and are in a Columbia (Col-0) background. The insertions are in the fourth exon and first intron, respectively (Figure 1). The *Atps1-3* (FLAG-456A09) insertion is from the Versailles T-DNA collection(SAMSON et al., Nucleic Acids Res, 30, 94-7, 2002) and is in a Wassilewskija (Ws-4) background, it is located in the second exon (Figure 1). RT-PCR was carried out using the pAtpsF/pAtpsR primers (Figure 1) on RNA from the *Atps1-3* and *Atps1-1* mutants and no detectable levels of the *AtPS1* transcript were amplified, indicating that these two alleles are null. When the same primers were used on RNA from the *Atps1-2* mutant normal expression levels of this region of the AtPS1 transcript were observed (data not shown). Nevertheless, the phenotype analysis described below strongly suggests that this third allele is also null.

### EXAMPLE 2: MEIOSIS IN Atps1 MUTANTS GENERATES DYADS AT HIGH FREQUENCY

In *A. thaliana,* the product of male meiosis is a group of four spores, organized in a tetrahedron, called tetrad. Our observation of the male meiotic products in wild type revealed almost exclusively tetrads (Figure 3). In rare occasions, (13/304) groups of three spores were also observed and certainly resulted from occasional superposition of spores. In contrast, the observation of meiotic products of the three independent *Atps1* mutants revealed the presence of a high frequency of dyads, triads and other uneven meiotic products (Figure 3A and 3B). The *Atps1-1* and *Atps1-2* mutants produced a majority of dyads (∼65%). The *Atps1-3* mutant phenotype appeared to be weaker and only 8% of its meiotic products were dyads. Complementation tests realized between *Atps1-1* and *Atps1-2* and between *Atps1-3* and *Atps1-1* showed that these mutations are allelic (Figure 3B), and thus demonstrated that the disruption of the *AtPS1* gene is responsible for the production of dyads observed in this series of mutant.

The *Atps1-3* mutant exhibited a weaker phenotype than the two other alleles, whereas expression analysis suggested that this allele is also null. As this allele was in a different genetic background (Ws-4) to the two others (Col-0), we tested if this difference could be influencing the strength of the phenotype by introducing the Col-0 mutation into the Ws-4 background and *vice versa.* As expected for a background effect, the frequency of dyads increased with successive backcrosses when *Atps1-3* was introduced into Col-0 (from 8% to 58% after four backcrosses) and decreased when *Atps1-1* was introduced into the Ws-4 background (from 64% to 13 % after four backcrosses). These results clearly indicate that the formation of diploid gametes is influenced by multiple genes, with *AtPS1* acting as a major gene.

### EXAMPLE 3: Atps1 MUTANTS PRODUCE VIABLE DIPLOID POLLEN GRAINS

Pollen grain viability was examined by Alexander staining (ALEXANDER, Stain Technol, 44, 117-22, 1969) and showed that in the majority of cases the dyads and triads produced by the mutants result in viable pollen grains (more than 95% in the different *Atps1* mutants: Col: 0 dead pollen grains out of 181; *Atps1-1:* 44 dead pollen grains out of 948; *Atps1-2:* 3 dead pollen grains out of 363). We did observe however that the pollen grains in mutant plants varied in size (data not shown). We then assessed the ploidy level of *Atps1-1* and *Atps1-2* pollen grains by quantifying spermatic nuclei DNA. Both mutants exhibited two different populations of pollen grains, one corresponding to viable haploid pollen grains (∼40% estimated by maximum likelihood) and another to viable diploid pollen grains (∼60% estimated) (data not shown). These proportions are compatible with the proportion of dyads, triads and tetrads observed in the mutants. In summary, the *Atps1-1* and *Atps1-2* mutants produce a high frequency of viable diploid pollen grains.

### EXAMPLE 4: SPONTANEOUS TRIPLOIDS APPEARS AMONG THE OFFSPRING OF DIPLOID alps1 MUTANTS

As *Atps1* mutants produce viable diploid pollen grains we search in the offspring of the diploid homozygous mutants the presence of polyploid plants by flow cytometry. Diploid and triploid plants (30%), but no tetraploid plants, were found among the progenies of *Atps1-1* and *Atps1-2* mutants (*Atps1-1:* 38 triploids out of 130 plants; *Atps1-2:* 30 triploids out of 103 plants). Flow cytometry results were confirmed by caryotyping a subset of 29 plants which were all confirmed to be triploid. This demonstrated that the diploid gametes produced in the *Atps1* mutants are involved in fertilisation and produce viable triploid plants. The appearance of triploid, but not tetraploids, suggests that the *Atps1* mutations only affect male meiosis.

To confirm that diploid gametes are produced only by the male side in *Atps1* mutants, we realized reciprocal crosses between *Atps1-2* mutants and wild type plants. As expected for the absence of a female meiotic defect we never isolated triploid plants when mutant ovules were fertilised with wild type pollen grains (0 triploids out of 182 plants). When mutant pollen was used for the cross we again observed that 30% of the progeny were triploids (20 triploids out of 56 plants).

The observed frequency of triploid plants (30%) among *Atps1-1* and *Atps1-* 2 mutant progeny is lower than expected from the frequency of diploid pollen grains produced by these mutants (∼60%). In parallel, more than 50% of the seeds obtained by selfing the *Atps1-1* and *Atps1-2* mutants, or backcrossing them as male, were abnormally coloured and shaped and germinated at a rate of ∼55%. Thus a potential explanation for the discrepancy between the frequency of diploid pollen grains and the frequency of triploid in the progeny, is abnormal seed development, which is commonly observed during crosses between plant species with different ploidy levels. These problems are related to the paternal to maternal ratio, which is very important for normal albumen development (SCOTT et al., Development, 125, 3329-41, 1998). The normal maternal to paternal ratio of albumen is 2:1 but in our case when a diploid sperm nucleus fertilised the two polar nuclei an aberrant 2:2 ratio was obtained. Nevertheless, it appears that approximately 25% of the triploid embryos were able to overcome this constraint.

### EXAMPLE 5: Atps1 MUTANTS ARE AFFECTED IN THE MEIOSIS II SPINDLES ORIENTATION

To unravel the mechanisms responsible of the production of dyads in *atps1-1*, we investigated the behavior of meiotic chromosomes in *atps1-1* mutant and in the wild type (Figure 4). Chromosome spreads showed that the meiosis in the *Atps1-1* mutant progresses normally and is indistinguishable from the wild type until the end of the telophase I. Synapsis was complete, chiasmata formed (the cytological manifestation of crossovers) and bivalents were seen (compare Figure 4G-I with Figure 4 A-C, for example). At metaphase II, however, differences were seen compared to wild type with the 10 chromosomes aligned in a same plane, causing abnormal looking Figures, rather than two well separated metaphase II plates containing five chromosomes each (Compare Figure 4J-K with Figure 4D). In rare cases, metaphase II in *Atps1* did appear normal however (Figure 4L). At telophase II, we observed dyads (two sets of 10 chromosomes, Figure 4M), triads (2 sets of five chromosomes and one set of 10, Figure 4N) and normal tetrads (4 sets of 5 chromosomes, Figure 4O). These observations are consistent with the previous finding that *Atps1* meiotic products are a mixture of dyads, triads and tetrads.

These results and specifically the alignment of the 10 chromosomes at metaphase II suggested that the meiotic spindles in *Atps1* mutants are defective at this stage. We thus examined spindle organisation by immunolocalisation with an alpha-tubulin antibody (Figure 5). In wild type plants the majority of metaphase II spindles were roughly perpendicular to each other (Figure 5A), leading to four well separated poles at anaphase II (Figure 5B) and the formation of tetrads (Figure 5C). In the *Atps1* mutant, while individual metaphase II / anaphase II spindles appeared regular in most cases their respective orientation was aberrant. The majority of cells had parallel spindles (Figure 5D to 5G), fused spindles (Figure 5H and 5I) or tripolar spindles (Figure 5J and 5K). This defect in spindle orientation explains the appearance of triads and dyads. These conformations cause chromatids, that had been separated at meiosis I, to gather at anaphase II. Occasionally, three to four sets of chromosomes encompassed by a spindle were dispersed in the cell at metaphase II (Figure 5L). This type of defect is probably the cause of the few unbalanced meiotic products observed in the *Atps1* mutants.

The name *AtPS1* for *Arabidopsis thaliana parallel spindles 1* was chosen due to the high percentage of parallel spindles produced by the corresponding mutants.

### EXAMPLE 6: DYADS PRODUCTION IN Atps1 MUTANT RESULTS FROM THE FORMATION OF PARALLEL SPINDLES AT METAPHASE II.

The apparition of parallel spindles at metaphase II in the *Atps1* mutants appear to be leading to the formation of dyads. This proposed mechanism implies that unbalanced chromosome segregation at meiosis I would have no impact on the final distribution of chromosomes in the resulting dyad. To test this hypothesis we constructed a double *Atspo11-1*/*Atps1* mutant.

The *Atspo11-1* mutant (N646172 *(Atspo11-1-3 :* STACEY et al., Plant Journal, 48, 206-16, 2006) obtained from the European Arabidopsis stock centre (SCHOLL et al., Plant Physiol, 124, 1477-80, 2000) displays an absence of bivalents at meiosis (MERCIER et al., Biochimie, 83, 1023-28, 2001) (Figure 6A) leading to frequent unbalanced first divisions (Figure 6B) that can be associated with lagging chromosomes (Figure 6C). At metaphase II, unbalanced plates are seen (Figure 6D), leading to unbalanced tetrads (Figure 6E). Lagging chromosomes at anaphase II, lead to multiple metaphase II plates and then polyads with more than four nuclei (Figure 6F).

In the *Atspo11-1*/*Atps1* background the first division was identical to the single *Atspo11-1* phenotype. We observed 10 univalents at metaphase I (Figure 6G), leading to missegregation at anaphase I, with two sets of unbalanced chromosomes (Figure 6H) or three sets because of lagging chromosomes (Figure 6I). At metaphase II, we regularly observed two unbalanced metaphase plates, which had a tendency to be parallel instead of perpendicular (Figure 6J). This led to the formation of dyads which were always balanced (Figure 6K to 6L, n=44). We also observed triads with one set of 10 chromosomes caused by an unbalanced first division followed by the fusion of two of the four second division products (Figure 6N), which is highly consistent with our proposed mechanism. We also observed unbalanced tetrads (Figure 6P), expected since the *Atps1* mutation is not fully penetrant, and polyads due to lagging chromosomes at the first division (Figure 6R).

Another prediction of the proposed mechanism is that centromere distribution should resemble that seen during mitosis, e.g. any heterozygosity at the centromeres should be retained in the diploid gametes. Indeed, in *Atps1*, the first division is identical to wild type, with the co-segregation of sister chromatids and separation of homologous chromatids. Thus, in the case of a heterozygous genotype, *A*/*a*, at the centromere, following the first division the two *A* alleles will end up at one pole, and the two *a* alleles at the opposite pole. In wild type, the second division separates the two sisters leading to four spores with one chromatid. In *Atps1,* the second division would regroup the products of the first division, thus grouping the *a* and *A* allele in each cell, leading to systematic heterozygosis at the centromere. Because of recombination, loci unlinked to centromeres should segregate randomly. We tested this prediction by taking advantage of the two genetic backgrounds of the *Atps1-1*(Col*-*0) and *Atps1-3* mutants (Ws-4). F1 plants bearing the two mutations - thus mutant for *AtPS1* and heterozygous for any Col-0/Ws-4 polymorphisms - were crossed as male to a third genetic background *landsberg erecta* (Ler). Caryotyping and genotyping of the obtained plants for trimorphic molecular markers (see additional methods) provided direct information regarding the genetic make up of the pollen grain produced by the mutant (Figure 7). All the diploid pollen grains tested had the predicted genetic characteristics. They were systematically heterozygous at centromeres and segregating - because of recombination- at other loci. These results confirm that the "parallel spindle" defect is indeed the cause of at least the vast majority of 2n pollen in *Atps1.*

### Conclusion:

The above results show that mutants in the *AtPS1* gene produce pollen grains which are up to 65% diploid and give rise to numerous triploid plants in the next generation. It is also shown *Atps1* mutations only affect male meiosis and result in abnormal orientation of spindles at meiosis II. Since defects in meiosis II spindles are the main source of the 2n pollen which is extensively used in many plant breeding programs (for example in potato cf. CARPUTO et al., Genetics, 163, 287-94, 2003), the identification of a gene involved in these defects has important applications in plant breeding.

### EXAMPLE 7: EXTINCTION OF ATPS1 BY RNAI PHENOCOPIES THE ATPS1 MUTANTS.

An RNA interference (RNAi) hairpin construct was made based on a 361-bp cDNA fragment of the *AtPS1* gene, represented as SEQ ID NO: 38.

The Prfb18 binary vector is derived from the pfgc5941 binary vector (GenBank: AY310901.1) by addition of GATEWAY® cloning sites on both sides of the chalcone synthase intron under control of the 35S promoter. The cDNA fragment SEQ ID NO: 38 and its reverse complement were placed in the GATEWAY® cloning sites of the Prb18 binary vector. The resulting vector comprises, downstream the 35S promoter, the reverse complement of SEQ ID NO: 38, followed by the chalcone synthase intron, followed by the cDNA fragment SEQ ID NO: 38, and by the octopine synthase polyA signal. This vector was used to transform *Arabidopsis* Col-0 wild type plants. The meiotic products of 14 primary transformants were observed. The results are summarized in Table I below.

**Table I**

| Genotype | % of dyads | % of triads | %tetrads |
|---|---|---|---|
| Col-0 | 0 | 0 | 100 |
| *atps1-1* | 64 | 24 | 11 |
| *atps1-2* | 65 | 20 | 13 |
| *RNAi 35S AtP51 #1* | 17 | 32 | 49 |
| *RNAi 35S ATPS1 #2* | 4 | 8 | 84 |
| *RNAi 35S AtPS1 #3* | 17 | 27 | 52 |
| *RNAi 35S AtPS1 #4* | 20 | 16 | 60 |
| *RNAi 35S AtPS1 #5* | 15 | 21 | 60 |
| *RNAi 35S AtPS1 #6* | 62 | 16 | 20 |
| *RNAi 35S AtPS1 #7* | 0 | 0 | 100 |
| *RNAi 35S AtPS1 #8* | 0 | 0 | 100 |
| *RNAi 35S AtPS1 #9* | 0 | 0 | 100 |
| *RNAi 35S AtPS1 #10* | 0 | 0 | 100 |
| *RNAi 35S AtPS1 #11* | 0 | 0 | 100 |
| *RNAi 35S AtPS1 #12* | 0 | 0 | 100 |
| *RNAi 35S AtPS1 #13* | 0 | 0 | 100 |
| *RNAi 35S AtPS1 #14* | 0 | 0 | 100 |

These results show that while wild type produces only tetrads of spores, *atps1* mutant and *RNAi 35SAtPS1* lines produce a high proportion of dyads.

Among the 14 *RNAi 35SAtPS1* lines, six produced a high frequency of dyads and triads instead of tetrads, a phenotype identical to the *Atps1* mutant. Figure 8 shows examples of meiotic products of a wild type plant, an *atps1-1* mutant and a plant of *RNAI35 AtPS1#6* line.

The level of expression of *AtPS1* in the Col-0 wild type plants was compared with the level of expression in three *RNAi 35SAtPS1* lines (*RNAi 35SAtPS1 #1, RNAi 35S AtPS1* #2, and *RNAi 35S AtPS1* #6), producing different proportions of dyads. RT-PCR were performed on flower buds with primers specific of *ATPS1:*
5' CCATAGTGAGAGTTATGGAGC 3' (SEQ ID NO: 39), and
5' GGCGCCTTTTCAACCTCTACTTG 3' (SEQ ID NO: 40)
or as a control, with primers specific of of the ubiquitous gene *APT.*

The results are shown on Figure 9. These results show that *AtPS1* expression is reduced in the three *RNAi 35SAtPS1* lines compared to the wild type (col).

Further there is a correlation between the efficiency of the *AtPS1* expression reduction, and the proportion of dyads (cf. Table I) produced by the *RNAi 35SAtPS1* lines.

Next, the ploidy level of the offspring of two *RNAi 35S AtPS1 lines* by flow cytometry was measured (results not shown). Triploids were found among the offspring of *RNAi 35S AtPS1#1 and RNAi 35S AtPS1#6.* This demonstrated that, like *Atps1* mutants, *RNAi 355 AtPS1 lines* can produce functional male diploid gametes.

## Claims

1. A method for obtaining a plant producing 2n pollen, wherein said method comprises the inhibition in said plant of a protein hereinafter designated as PS1 protein, wherein said protein:
- comprises within its N-terminal region (preferably within its 300 N-terminal amino-acids, more preferably within its 250 N-terminal aminoacids, and still more preferably within its 200 N-terminal aminoacids), a domain having at least 40%, and by order of increasing preference, at least 45, 50, 55, 60, 65, 70, 75, 80, 85, 90, 95 or 98% sequence identity, or at least 60%, and by order of increasing preference, at least, 65, 70, 75, 80, 85, 90, 95 or 98% sequence similarity with the FHA domain of the AtPS1 protein (amino acids 64-132 of SEQ ID NO: 1);
- comprises within its C-terminal region (preferably within its 350 C-terminal amino-acids, more preferably within its 300 C-terminal aminoacids, and still more preferably within its 250 C-terminal aminoacids), a domain having at least 40%, and by order of increasing preference, at least 45, 50, 55, 60, 65, 70, 75, 80, 85, 90, 95 or 98% sequence identity, or at least 60%, and by order of increasing preference, at least, 65, 70, 75, 80, 85, 90, 95 or 98% sequence similarity with the PINc domain of the AtPS1 protein (amino acids 1237-1389 of SEQ ID NO: 1).

2. A method according to claim 1, wherein inhibition of said protein is obtained by mutagenesis of the PS1 gene or of its promoter, and the mutants having partially or totally lost the PS1 protein activity are selected.

3. A method according to claim 1, wherein the inhibition of said protein is obtained by expressing in said plant of a silencing RNA targeting the gene encoding said protein.

4. An expression cassette comprising:
- a promoter functional in a plant cell;
- a DNA construct selected among:
a) a DNA construct of 200 to 1000 bp, preferably of 300 to 900 bp, comprising a fragment of a cDNA encoding a PS1 protein or of its complementary, or having at least 95% identity, and by order of increasing preference, at least 96%, 97%, 98%, or 99 % identity with said fragment
b) a DNA construct which is capable, when transcribed, of forming a hairpin RNA targeting the *PS1* gene;
c) a DNA construct which is capable, when transcribed, of forming an amiRNA targeting the *PS1* gene;
said DNA sequence being placed under transcriptional control of said promoter.

5. A recombinant vector comprising an expression cassette of claim 4.

6. A transgenic plant producing 2n pollen, wherein said plant contains a transgene comprising an expression cassette of claim 4.

7. A method for producing 2n pollen, wherein said method comprises:
- obtaining a plant producing 2n pollen, by a method of any of claims 1 to 3;
- cultivating said plant;
- recovering the pollen produced by said plant.

## Patentansprüche

1. Verfahren zum Erhalten einer Pflanze, die 2n-Pollen herstellt, wobei das Verfahren die Inhibition eines Proteins, hierin im Folgenden bezeichnet als PS1-Protein, in der Pflanze umfasst, wobei das Protein:
- innerhalb seiner N-terminalen Region (bevorzugt seiner 300 N-terminalen Aminosäuren, bevorzugter innerhalb seiner 250 N-terminalen Aminosäuren, und noch bevorzugter innerhalb seiner 200 N-terminaler Aminosäuren) eine Domäne umfasst, die mindestens 40%, und in der Reihenfolge steigender Präferenz mindestens 45, 50, 55, 60, 70, 75, 80, 85, 90, 95 oder 98% Sequenzidentität hat, oder mindestens 60%, und in der Reihenfolge steigender Präferenz mindestens 65, 70, 75, 80, 85, 90, 95 oder 98% Sequenzähnlichkeit mit der FHA-Domäne des AtPS1-Proteins (Aminosäuren 64-132 von SEQ ID NO: 1) hat;
- innerhalb seiner C-terminalen Region (bevorzugt innerhalb seiner 350 C-terminalen Aminosäuren, bevorzugter innerhalb seiner 300 C-terminalen Aminosäuren und noch bevorzugter innerhalb seiner 250 C-terminalen Aminosäuren) eine Domäne umfasst, die mindestens 40%, und in der Reihenfolge steigender Präferenz mindestens 45, 50, 55, 60, 70, 75, 80, 85, 90, 95 oder 98% Sequenzidentität hat, oder mindestens 60%, und in der Reihenfolge steigender Präferenz mindestens 65, 70, 75, 80, 85, 90, 95 oder 98% Sequenzähnlichkeit mit der PINc-Domäne des AtPS1-Proteins (Aminosäuren 1237-1389 von SEQ ID NO: 1) hat.

2. Verfahren gemäß Anspruch 1, wobei Inhibition des Proteins durch Mutagenese des PS1-Gens, oder seines Promotors, erhalten wird und die Mutanten, die teilweisen oder totalen Verlust der PS1-Proteinaktivität haben, ausgewählt werden.

3. Verfahren gemäß Anspruch 1, wobei die Inhibition des Proteins durch Exprimieren einer Silencing-RNA, die das Gen targetiert, das das Protein codiert, in der Pflanze erhalten wird.

4. Expressionskassette, umfassend:
- einen Promotor, der in einer Pflanzenzelle funktional ist;
- ein DNA-Konstrukt, ausgewählt aus:
a) einem DNA-Konstrukt von 200 bis 1000 bp, bevorzugt von 300 bis 900 bp, umfassend ein Fragment einer cDNA, die ein PS1-Protein codiert, oder ihres Komplements, oder die mindestens 95% Identität, und in der Reihenfolge aufsteigender Präferenz mindestens 96%, 97%, 98% oder 99% Identität mit dem Fragment hat;
b) einem DNA-Konstrukt, das dazu in der Lage ist, eine Haarnadel-RNA zu bilden, die das *PS1*-Gen targetiert, wenn es transkribiert wird;
c) einem DNA-Konstrukt, das dazu in der Lage ist, eine amiRNA, die das *PS1*-Gen targetiert, zu bilden, wenn es transkribiert wird;
wobei die DNA-Sequenz unter transkriptionelle Kontrolle des Promotors platziert wird.

5. Rekombinanter Vektor, der eine Expressionskassette gemäß Anspruch 4 umfasst.

6. Transgene Pflanze, die 2n-Pollen herstellt, wobei die Pflanze ein Transgen enthält, das eine Expressionskassette gemäß Anspruch 4 umfasst.

7. Verfahren zur Herstellung von 2n-Pollen, wobei das Verfahren umfasst:
- Erhalten einer Pflanze, die 2n-Pollen herstellt, durch ein Verfahren gemäß einem beliebigen der Ansprüche 1 bis 3;
- Kultivieren der Pflanze;
- Gewinnen der Pollen, die von der Pflanze hergestellt wurden.

## Revendications

1. Procédé d'obtention d'une plante produisant du pollen 2n, dans lequel ledit procédé comprend l'inhibition dans ladite plante d'une protéine désignée ci-après par protéine PS1, dans laquelle ladite protéine :
- comprend au sein de sa région N-terminale (de préférence au sein de ses 300 acides aminés N-terminaux, de manière davantage préférée au sein de ses 250 acides aminés N-terminaux, ou mieux au sein de ses 200 acides aminés N-terminaux), un domaine présentant au moins 40 %, et par ordre de préférence croissante, au moins 45, 50, 55, 60, 65, 70, 75, 80, 85, 90, 95 ou 98 % d'identité de séquence, ou au moins 60 %, et par ordre de préférence croissante, au moins 65, 70, 75, 80, 85, 90, 95 ou 98 % de similarité de séquence avec le domaine FHA de la protéine AtPS1 (acides aminés 64 à 132 de SEQ ID NO : 1) ;
- comprend au sein de sa région C-terminale (de préférence au sein de ses 350 acides aminés C-terminaux, de manière davantage préférée au sein de ses 300 acides aminés C-terminaux, ou mieux au sein de ses 250 acides aminés C-terminaux), un domaine présentant au moins 40 %, et par ordre de préférence croissante, au moins 45, 50, 55, 60, 65, 70, 75, 80, 85, 90, 95 ou 98 % d'identité de séquence, ou au moins 60 %, et par ordre de préférence croissante, au moins 65, 70, 75, 80, 85, 90, 95 ou 98 % de similarité de séquence avec le domaine PINc de la protéine AtPS1 (acides aminés 1237 à 1389 de SEQ ID NO : 1).

2. Procédé selon la revendication 1, dans lequel l'inhibition de ladite protéine est obtenue par la mutagenèse du gène de PS1 ou de son promoteur, et les mutants ayant perdu partiellement ou totalement l'activité de la protéine PS1 sont sélectionnés.

3. Procédé selon la revendication 1, dans lequel l'inhibition de ladite protéine est obtenue par l'expression dans ladite plante d'un ARN silencieux ciblant le gène codant ladite protéine.

4. Cassette d'expression comprenant :
- un promoteur fonctionnel dans une cellule végétale ;
- une construction d'ADN choisie parmi :
a) une construction d'ADN contenant 200 pb à 1000 pb, de préférence 300 pb à 900 pb, et comprenant un fragment d'ADNc codant une protéine PS1 ou de son brin complémentaire, ou présentant au moins 95 % d'identité, et par ordre de préférence croissante, au moins 96 %, 97 %, 98 % ou 99 % d'identité avec ledit fragment ;
b) une construction d'ADN qui est capable, quand elle est transcrite, de former un ARN en épingle à cheveux ciblant le gène *PS1 ;*
c) une construction d'ADN qui est capable, quand elle est transcrite, de former un micro-ARN artificiel ciblant le gène *PS1 ;*
ladite séquence d'ADN étant placée sous le contrôle transcriptionnel dudit promoteur.

5. Vecteur recombinant comprenant une cassette d'expression selon la revendication 4.

6. Plante transgénique produisant un pollen 2n, dans laquelle ladite plante contient un transgène comprenant une cassette d'expression selon la revendication 4.

7. Procédé de production d'un pollen 2n, dans lequel ledit procédé comprend :
- l'obtention d'une plante produisant un pollen 2n par un procédé selon l'une quelconque des revendications 1 à 3 ;
- la culture de ladite plante ;
- la récupération du pollen produit par ladite plante.
